# EUROPÄISCHE PATENTSCHRIFT

(11) **EP 4 057 932 B1**
(45) Veröffentlichungstag und Bekanntmachung des Hinweises auf die Patenterteilung: **29.10.2025**
(21) Anmeldenummer: 21814710.6
(22) Anmeldetag: 11.11.2021
(51) Int. Cl.: A61B 50/30, A61B 50/34, A61B 50/00, B65D 65/12

(54) **MEDIZINISCHE VERPACKUNGSVORRICHTUNG UND VERFAHREN ZUM HERSTELLEN EINER MEDIZINISCHEN VERPACKUNGSVORRICHTUNG ZUM STERILEN VERPACKEN EINES SIEBKORBES**
MEDICAL PACKAGING DEVICE AND METHOD FOR PRODUCING A MEDICAL PACKAGING DEVICE FOR STERILE PACKING OF A STRAINER BASKET
DISPOSITIF D'EMBALLAGE MÉDICAL ET PROCÉDÉ DE PRODUCTION D'UN DISPOSITIF D'EMBALLAGE MÉDICAL POUR EMBALLAGE STÉRILE D'UN PANIER-FILTRE

(30) Priorität: 07.12.2020 DE 102020132419
(43) Veröffentlichungstag der Anmeldung: 21.09.2022
(73) Patentinhaber: Aesculap AG, 78532 Tuttlingen (DE)
(72) Erfinder: HENKE, Matthias, 78048 Villingen-Schwenningen (DE); BOHNENSTENGEL, Philipp, 78256 Steisslingen (DE)
(74) Vertreter: Hoeger, Stellrecht & Partner Patentanwälte mbB
(86) Internationale Anmeldenummer: PCT/EP2021/081330
(87) Internationale Veröffentlichungsnummer: WO 2022/122290

(56) Entgegenhaltungen:
- EP-A1- 2 992 851
- AU-B2- 2015 224 419
- JP-A- H07 215 369
- US-A- 2 265 680
- US-A- 3 680 772
- US-A- 4 705 171
- US-A- 5 447 230
- US-A1- 2002 092 274
- US-A1- 2007 026 472

## Beschreibung

Die vorliegende Erfindung betrifft eine medizinische Verpackungsvorrichtung zum sterilen Verpacken eines Siebkorbs, wobei die Verpackungsvorrichtung einen Aufnahmeraum zum Aufnehmen eines Siebkorbs in einer Verpackungsstellung und eine Einführöffnung zum Einführen eines Siebkorbs in den Aufnahmeraum hinein definiert, wobei die Einführöffnung in einer Einführstellung offen ist, wobei die Verpackungsvorrichtung aus einem in einer Ausgangsstellung ungefalteten Flachmaterialbogen durch mehrfaches Falten gebildet ist, wobei der Aufnahmeraum in der Einführstellung zwei aneinander anliegende Aufnahmeraumflachmaterialbogenflächenbereiche aufweist, welche durch mindestens drei Faltlinien des Flachmaterialbogens begrenzt sind derart, dass der Aufnahmeraum bis auf die Einführöffnung allseitig geschlossen ist.

Ferner betrifft die vorliegende Erfindung ein medizinisches Verpackungssystem für Siebkörbe.

Überdies betrifft die vorliegende Erfindung ein Verfahren zum Herstellen einer medizinischen Verpackungsvorrichtung zum sterilen Verpacken eines Siebkorbs, wobei die Verpackungsvorrichtung ausgebildet wird mit einem Aufnahmeraum zum Aufnehmen eines Siebkorbs in einer Verpackungsstellung und einer Einführöffnung zum Einführen eines Siebkorbs in den Aufnahmeraum hinein, wobei die Einführöffnung in einer Einführstellung offen ist, wobei die Verpackungsvorrichtung aus einem in einer Ausgangsstellung ungefalteten Flachmaterialbogen durch mehrfaches Falten gebildet wird, wobei der Aufnahmeraum in der Einführstellung zwei aneinander anliegende Aufnahmeraumflachmaterialbogenflächenbereiche aufweist, welche durch mindestens drei Faltlinien des Flachmaterialbogens begrenzt sind derart, dass der Aufnahmeraum bis auf die Einführöffnung allseitig geschlossen ist.

Siebkörbe oder auch Sterilisiersiebschalen werden nach dem Beschicken mit Instrumenten oder Implantaten üblicherweise in Vliestücher eingeschlagen, bevor sie sterilisiert werden. Hierfür werden große Vliestücher benötigt. Nach dem Einschlagen in ein Vliestuch ist ein Siebkorb somit von einer Weichverpackung, gebildet durch das Vliestuch, umgeben. In einem nächsten Schritt werden die so verpackten Siebkörbe in einen Sterilisierbehälter eingebracht und in diesem sterilisiert, insbesondere heißdampfsterilisiert.

Der Aufwand, um Siebkörbe in der beschriebenen Weise mit einer Weichverpackung zu umgeben, ist sehr groß. Die in Form von Bögen bereitgestellten Vliestücher müssen insbesondere beschädigungsfrei gelagert werden. Ferner müssen Mitarbeiter in unterschiedliche Falttechniken eingewiesen werden, um den Siebkorb vollständig in das Vliestuch einzuschlagen. Dabei kann es immer wieder zu falsch verpackten Siebkörben kommen, was insbesondere mit der Gefahr verbunden ist, dass die gewünschte Sterilität des Siebkorbs und seines Inhalts nicht in jedem Fall gegeben ist.

Bekannt ist es insbesondere, Siebkörbe in eine Diagonalverpackung oder eine Parallelverpackung gemäß DIN 58953-7 einzuschlagen. Hierfür sind viele händische Faltschritte erforderlich, um den Siebkorb korrekt in das Vliestuch einzuschlagen. Damit einhergehend verbunden ist ein hoher Schulungsaufwand für das den Verpackungsschritt durchführende Personal. Unabhängig davon hängt die Qualität der Verpackung des Siebkorbs letztlich vom jeweiligen Mitarbeiter ab. Aufgrund des hohen Zeitaufwands entstehen für die Verpackung des Siebkorbs hohe Kosten.

In der US 2007/0026472 A1 ist eine Sterilhülle mit zusätzlichem Verstärkungsbogen beschrieben. Aus der US 2002/0092274 A1 sind Verfahren und Verpackungssysteme zum Verpacken eines sterilisierten Gegenstands bekannt.

Die AU 2015/224419 B2 befasst sich mit flexiblen, mehrere Beschriftungslappen aufweisenden Sterilanordnungen. Aus der EP 2 992 851 A1 sind ein Verpackungssystem für aseptisch zu präsentierende medizinische Instrumente sowie ein Verfahren unter Verwendung des Verpackungssystems bekannt. Die US 5,447,230 betrifft Verpackungen für chirurgische Instrumente. Eine Sterilverpackung ist in der JP H07 215369 A beschrieben. Eine versiegelte Behälteranordnung ist in der US 3,680,772 offenbart. Aus der US 2,265,680 ist Sterilisierbehältnis bekannt. Ein Umschlag für sterile Einwegartikel ist in der US 4,705,171 beschrieben.

Es ist daher eine Aufgabe der vorliegenden Erfindung, eine medizinische Verpackungsvorrichtung, ein medizinisches Verpackungssystem, ein Verfahren zum Herstellen einer medizinischen Verpackungsvorrichtung und ein Verfahren zum sterilen Verpacken eines Siebkorbs zu verbessern.

Diese Aufgabe wird bei einer medizinischen Verpackungsvorrichtung der eingangs beschriebenen Art erfindungsgemäß dadurch gelöst, dass die Verpackungsvorrichtung eine Entfaltungssicherungseinrichtung umfasst zum Sichern der Verpackungsvorrichtung in der Einführstellung gegen ein vollständiges Entfalten zurück in die Ausgangsstellung.

Die vorgeschlagene medizinische Verpackungsvorrichtung zum sterilen Verpacken eines Siebkorbs ermöglicht es einem Anwender insbesondere, den Siebkorb durch die Einführöffnung in den Aufnahmeraum einzubringen, insbesondere einzuschieben. Die in der Einführstellung bereitgestellte medizinische Verpackungsvorrichtung hat dann insbesondere den Vorteil, dass ein wesentlicher Teil der sonst üblicherweise vorzunehmenden Faltungen des Flachmaterialbogens durch den Anwender, wie es die oben genannte DIN-Norm vorsieht, entfällt. Der Anwender muss nach dem Einführen des Siebkorbs in den Aufnahmeraum lediglich die Einführöffnung verschließen, und zwar beispielsweise durch Umschlagen eines freien Endes der Verpackungsvorrichtung über die Einführöffnung. Ferner ermöglicht es die Entfaltungssicherungseinrichtung die medizinische Verpackungsvorrichtung in der Einführstellung zu sichern. Beispielsweise können durch die Entfaltungssicherungseinrichtung umgeschlagene Laschen oder Umschläge der Verpackungssicherungseinrichtung fixiert werden. So kann insbesondere verhindert werden, dass die Verpackungsvorrichtung wieder vollständig zurück in die Ausgangsstellung entfaltet werden kann. Die bereits durch das Falten vorkonfektionierte Verpackungsvorrichtung wird durch die Entfaltungssicherungseinrichtung zusätzlich gesichert, wodurch sich eine Handhabung der Verpackungsvorrichtung zum sterilen Verpacken eines Siebkorbs für einen Anwender weiter verbessert. Eine Sicherung kann insbesondere durch Kleben mit einem Klebstoff, beispielsweise Cyanacrylaten oder Schmelzklebern, durch ein doppelseitiges Klebeband, durch Ultraschallverschweißen oder durch einen aufgeklebten Klettverschluss erfolgen.

Günstig ist es, wenn der der Flachmaterialbogen eine erste Flachmaterialseitenfläche und eine zweite Flachmaterialseitenfläche definiert, wenn die erste Flachmaterialseitenfläche und die zweite Flachmaterialseitenfläche in der Ausgangsstellung in voneinander entgegengesetzte Richtungen weisen und wenn die erste Flachmaterialseitenfläche die zwei Aufnahmeraumflachmaterialbogenflächenbereiche umfasst. Auf diese Weise kann insbesondere sichergestellt werden, dass der Siebkorb beim Verpacken mit der Verpackungsvorrichtung nur mit einer der beiden Flachmaterialseitenflächen des Flachmaterialbogens in Kontakt kommen kann, nämlich mit der ersten Flachmaterialseitenfläche. So kann ein Kontakt und damit auch eine Kontamination des Siebkorbs mit der zweiten Flachmaterialseitenfläche vermieden werden.

Vorteilhaft ist es, wenn die Verpackungsvorrichtung in der Einführstellung mindestens drei Faltlinien umfasst. Insbesondere kann sie drei, vier, fünf, sechs oder sieben Faltlinien umfassen. Je mehr Faltlinien die Verpackungsvorrichtung bereits umfasst, umso weniger Faltungen muss ein Anwender gegebenenfalls noch vornehmen, nachdem er den Siebkorb in den Aufnahmeraum der Verpackungsvorrichtung eingebracht hat. So kann die vorkonfektionierte Verpackungsvorrichtung durch die Anzahl der Faltlinien unterschiedliche Vorkonfektionierungsstufen aufweisen, je nachdem, wieviel Unterstützung ein Anwender bei der Verpackung von Sterilbehältern benötigt.

Auf einfache Weise lässt sich die Verpackungsvorrichtung ausbilden, wenn der Flachmaterialbogen in der Ausgangsstellung viereckig ist. Insbesondere kann er rechteckig ausgebildet sein. Vorzugsweise ist der Flachmaterialbogen quadratisch oder im Wesentlichen quadratisch ausgebildet. Dies ermöglicht es insbesondere, definierte Faltlinien auf einfache Weise zu erzeugen, beispielsweise diagonale Faltlinien, die einander gegenüberliegenden Ecken des Flachmaterialbogens miteinander verbinden.

Günstig ist es, wenn eine der mindestens drei Faltlinien in Form einer Hauptfaltlinie ausgebildet ist und wenn die Hauptfaltlinie zwei in der Ausgangsstellung einander gegenüberliegende Hauptecken des Flachmaterialbogens miteinander verbindet. Ist der Flachmaterialbogen quadratisch, wird er durch die Hauptfaltlinie in zwei identische Dreiecke aufgeteilt, die nach einer ersten Faltung um die Hauptfaltlinie aufeinanderliegen.

Die Verpackungsvorrichtung lässt sich auf einfache Weise handhaben, wenn sich die Hauptfaltlinie parallel oder im Wesentlichen parallel zur Einführöffnung erstreckt.

Günstig ist es, wenn zwei der mindestens drei Faltlinien in Form von parallel oder im Wesentlichen parallel zueinander verlaufenden Seitenfaltlinien ausgebildet sind und wenn sich die Seitenfaltlinien quer zur Hauptfaltlinie erstrecken. Insbesondere können sich die Seitenfaltlinien senkrecht zur Hauptfaltlinie erstrecken. Auf diese Weise kann der Aufnahmeraum durch die Hauptfaltlinie und die zwei Seitenfaltlinien begrenzt werden. Abstände der beiden Seitenfaltlinien definieren somit insbesondere auch eine Größe, beispielsweise eine Breite, des Aufnahmeraums.

Günstigerweise entspricht ein Abstand der beiden Seitenfaltlinien voneinander mindestens einem Drittel des Abstands der Hauptecken voneinander. Auf diese Weise lässt sich insbesondere verhindern, dass die Hauptecken über die jeweils andere Seitenfaltlinie hinausragen und dann beim Umschlagen der anderen Hauptecke um die andere Seitenfaltlinie nochmals umgeschlagen werden müssen. Durch die vorgeschlagene Abstandsvorgabe kann also insbesondere eine besonders flache Verpackungsvorrichtung in der Einführstellung bereitgestellt werden.

Vorzugsweise sind die Hauptecken in der Einführstellung auf der Hauptfaltlinie oder im Wesentlichen auf der Hauptfaltlinie positioniert. So lassen sich die Seitenfaltlinien auf einfache Weise senkrecht zur Hauptfaltlinie ausbilden.

Um den Aufnahmeraum in gewünschter Weise vorgeben zu können, ist es vorteilhaft, wenn die zwei Aufnahmeraumflachmaterialbogenflächenbereiche durch die Hauptfaltlinie begrenzt sind.

Gemäß einer weiteren bevorzugten Ausführungsform der Erfindung kann vorgesehen sein, dass der Flachmaterialbogen in der Ausgangsstellung zwei einander gegenüberliegende Nebenecken umfasst, dass die zwei Nebenecken in einer ersten Faltstellung, in welcher der Flachmaterialbogen von der Ausgangsstellung um die Hauptfaltlinie gefaltet ist, aufeinander liegen, dass die Verpackungsvorrichtung eine erste Löselasche umfasst, dass die erste Löselasche durch Zurückfalten einer ersten der zwei Nebenecken aus der ersten Faltstellung in eine zweite Faltstellung um eine Nebenfaltlinie ausgebildet ist und dass die Nebenfaltlinie parallel oder im Wesentlichen parallel zur Hauptfaltlinie verläuft und sich zwischen der Hauptfaltlinie und den Nebenecken in der ersten Faltstellung erstreckt. Diese Ausgestaltung ermöglicht es insbesondere, eine der beiden Nebenecken, in diesem Fall die erste Nebenecke, als Teil einer Löselasche zu nutzen. Mit dieser ersten Löselasche kann ein Anwender die Verpackungsvorrichtung von einem Siebkorb auf einfache Weise lösen. Er muss lediglich die erste Löselasche mit der ersten Nebenecke erfassen und daran ziehen. So kann er die Verpackungsvorrichtung schnell und sicher vom Siebkorb entfalten.

Die Handhabung der Verpackungsvorrichtung kann auf einfache Weise verbessert werden, wenn die erste Löselasche in der Einführstellung über die Hauptfaltlinie vorsteht. So steht beispielsweise ein Zipfel mit der ersten Nebenecke über die Hauptfaltlinie vor, sodass ein Anwender diesen Zipfel zum Öffnen der Verpackungsvorrichtung schnell und sicher ergreifen kann.

Günstigerweise ist ein Abstand der Nebenfaltlinie von der Hauptfaltlinie kleiner als ein Abstand von den Nebenecken in der ersten Faltstellung. Durch diese Abstandvorgabe kann insbesondere erreicht werden, dass die erste Löselasche in der Einführstellung über die Hauptfaltlinie vorsteht.

Vorzugsweise begrenzt die Nebenfaltlinie die Einführöffnung. Zum Einführen des Siebkorbs muss dann lediglich die Nebenfaltlinie etwas angehoben werden, so dass die beiden in der Einführstellung aneinander anliegenden Aufnahmeraumflachmaterialbogenflächenbereiche etwas voneinander weg bewegt werden, so dass der Siebkorb einfach und sicher in den Aufnahmeraum eingeschoben werden kann. Die Nebenfaltlinie erleichtert einem Anwender das Einführen des Siebkorbs in den Aufnahmeraum, da sie erkennbar die Einführöffnung begrenzt.

Günstig ist es, wenn eine erste der zwei Hauptecken von der zweiten Faltstellung um eine erste der zwei Seitenfaltlinien in Richtung auf eine zweite der zwei Hauptecken hin in eine dritte Faltstellung umgefaltet ist und wenn die zweite der zwei Hauptecken von der dritten Faltstellung in eine vierte Faltstellung um eine zweite der zwei Seitenfaltlinien in Richtung auf die erste der zwei Seitenfaltlinien hin umgefaltet ist. Durch diese Ausgestaltung kann insbesondere ein rechteckiger Aufnahmeraum für einen Siebkorb definiert werden. Diese sind in der Regel ebenfalls rechteckig, beispielsweise auch quadratisch.

Ferner wird der Aufnahmeraum durch die zwei Seitenfaltlinien seitlich in definierter Weise verschlossen.

Einfach und kompakt ausbilden lässt sich die Verpackungsvorrichtung, wenn die vierte Faltstellung die Einführstellung definiert.

Ferner kann es vorteilhaft sein, wenn die Verpackungsvorrichtung eine zweite Löselasche umfasst, wenn die zweite Löselasche ausgebildet ist durch Umfalten einer zweiten der zwei Nebenecken, welche in der Einführstellung von der Einführöffnung weg weist, von der vierten Faltstellung in eine fünfte Faltstellung um eine Löselaschenhauptfaltlinie in Richtung auf die Hauptfaltlinie hin und durch Zurückfalten der zweiten der zwei Nebenecken von der fünften Faltstellung in eine sechste Faltstellung um eine Löselaschennebenfaltlinie in einer Richtung von der Hauptfaltlinie weg. So lässt sich insbesondere ein zweiter, nach Einführen des Sterilbehälters in den Aufnahmeraum von der Verpackungsvorrichtung abstehender Zipfel ausbilden, welcher die zweite Nebenecke umfasst. Ergreift ein Anwender diesen Zipfel, kann er die Verpackungsvorrichtung in einfacher und definierter Weise vom Siebkorb lösen, insbesondere entfalten.

Vorzugsweise ist ein Abstand der Löselaschenhauptfaltlinie von der Hauptfaltlinie größer als ein Abstand von den Nebenecken in der ersten Faltstellung. Insbesondere ist es günstig, wenn der Abstand der Löselaschenhauptfaltlinie von der Hauptfaltlinie mehr als doppelt so groß wie der Abstand der Löselaschenhauptfaltlinie von den Nebenecken in der ersten Faltstellung. So kann insbesondere sichergestellt werden, dass die zweite Nebenecke in der sechsten Faltstellung über die Löselaschenhauptfaltlinie und damit von der Verpackungsvorrichtung insgesamt vorsteht.

Vorteilhaft ist es, wenn ein Abstand der Löselaschennebenfaltlinie von der Lösenlaschenhauptfaltlinie größer ist als ein Abstand von der zweiten Nebenecke, insbesondere mindestens 30% größer. So kann insbesondere ein hinreichend großer Zipfel ausgebildet werden, welcher als zweite Löselasche fungieren kann für einen Anwender, um die Verpackungsvorrichtung sicher und definiert von einer Sterilisiersiebschale zu entfernen.

Vorteilhaft ist es, wenn die Löselaschenhauptfaltlinie und die Löselaschennebenfaltlinie parallel oder im Wesentlichen parallel zueinander verlaufen. Insbesondere können sie parallel zur Hauptfaltlinie verlaufen. Diese Ausgestaltung ermöglicht es insbesondere, eine rechteckige beziehungsweise nach Einführen eines Siebkorbs in den Aufnahmeraum quaderförmige beziehungsweise im Wesentlichen quaderförmige Verpackungsvorrichtung zu realisieren.

Vorteilhafterweise ist die Einführöffnung in der sechsten Faltstellung offen. Dies ermöglicht es insbesondere, die Verpackungsvorrichtung als vorkonfektionierte Verpackungsvorrichtung in der sechsten Faltstellung bereitzustellen. Ein Anwender kann dann in diese so vorgefertigte Verpackungsvorrichtung einen Siebkorb direkt einschieben und muss dann nur noch das von der Einführöffnung weg weisende Ende mit der zweiten Nebenecke über die Einführöffnung umschlagen, um den im Aufnahmeraum aufgenommenen Siebkorb vollständig zu umschließen.

Günstig ist es, wenn die Entfaltungssicherungseinrichtung mindestens ein Sicherungselement umfasst zum Sichern der Verpackungsvorrichtung in der Einführstellung. Insbesondere können mehrere Sicherungselemente vorgesehen sein, beispielsweise zwei, drei, vier oder mehr, um die vorkonfektionierte Verpackungsvorrichtung in der Einführstellung gegen ein Entfalten zu sichern.

Vorteilhaft ist es, wenn die Verpackungsvorrichtung mindestens einen ersten Sicherungsflächenbereich und mindestens einen zweiten Sicherungsflächenbereich definiert, wenn der erste Sicherungsflächenbereich und der zweite Sicherungsflächenbereich von den zwei in der Einführstellung aneinander anliegenden Aufnahmeraumflachmaterialbogenflächenbereichen verschieden sind, wenn der erste Sicherungsflächenbereich und der zweite Sicherungsflächenbereich in der Einführstellung aneinander anliegen und wenn das mindestens eine Sicherungselement den mindestens einen ersten Sicherungsflächenbereich und den mindestens einen zweiten Sicherungsflächenbereich kraft- und/oder stoffschlüssig miteinander verbindet, insbesondere durch Kleben und/oder Schweißen. In der beschriebenen Weise ist es also insbesondere möglich, Sicherungsflächenbereiche in definierter Weise miteinander zu verbinden. Insbesondere ist ausgeschlossen, dass die Sicherungsflächenbereiche durch die Aufnahmeraumflachmaterialbogenflächenbereiche definiert werden. So wird insbesondere verhindert, dass der Aufnahmeraum verkleinert wird. Sicherungsflächenbereiche können insbesondere definiert werden durch Flächenbereiche, die nach einem Umfalten längs einer Faltlinie aufeinander zu liegen kommen. Werden diese Flächen miteinander verbunden durch ein Sicherungselement oder mehrere Sicherungselemente, kann die Verpackungsvorrichtung insbesondere nicht mehr von selbst oder unabsichtlich in die Ausgangsstellung durch Entfalten überführt werden.

Um unterschiedlichste Flächen nach Faltungen des Flachmaterialbogens miteinander zu verbinden um sie aneinander zu sichern, ist es vorteilhaft, wenn der mindestens eine erste Sicherungsflächenbereich und der zugeordnete mindestens eine zweite Sicherungsflächenbereich von derselben Flachmaterialseitenfläche oder von unterschiedlichen Flachmaterialseitenflächen umfasst sind. Beispielsweise können Sicherungsflächenbereiche nahe der Hauptecken auf derselben Flachmaterialseitenfläche, nämlich der zweiten Flachmaterialseitenfläche, definiert sein.

Auf einfache Weise lässt sich die Verpackungsvorrichtung ausbilden, wenn das mindestens eine Sicherungselement durch einen Schweißpunkt, durch einen Klebstoff oder durch ein Klebeelement ausgebildet ist. Insbesondere kann das Klebeelement zwei voneinander weg weisende Klebeflächen aufweisen, wobei in der Einführstellung die eine der zwei Klebeflächen am mindestens einen ersten Sicherungsflächenbereich anliegt und wobei die andere der zwei Klebeflächen am mindestens einen zweiten Sicherungsflächenbereich anliegt. Das Klebeelement kann also beispielsweise in Form eines doppelseitigen Klebebandstreifens ausgebildet sein. Beispielsweise kann ein solches Klebeelement bereits auf dem ungefalteten Flachmaterialbogen, also in der Ausgangsstellung, an derjenigen Stelle oder Position aufgebracht sein, wo es später benötigt wird, um mit einem zweiten Sicherungsflächenbereich verbunden zu werden.

Günstig ist es, wenn das mindestens eine Sicherungselement in einem Bereich der Verpackungsvorrichtung positioniert ist, in welchem in der Einführstellung mindestens zwei durch Umfalten des Flachmaterialbogens ausgebildete Lagen aufeinanderliegen. Insbesondere kann das mindestens eine Sicherungselement in Bereichen positioniert sein, in denen drei, vier, fünf, sechs oder noch mehr Lagen des Flachmaterialbogens aufeinander liegen. Vorzugsweise wird der letzte Umschlag um eine der beiden Seitenfaltlinien mit einem Sicherungselement fixiert, beispielsweise auf dem gebildeten Umschlag um die andere der beiden Seitenfaltlinien.

Gemäß einer weiteren bevorzugten Ausführungsform der Erfindung kann vorgesehen sein, dass die die Verpackungsvorrichtung mindestens ein Verschlusselement umfasst zum Verschließen der Verpackungsvorrichtung in einer Verpackungsstellung, in welcher die Einführöffnung verschlossen ist durch Umfalten der zweiten Nebenecke um eine Verschlussfaltlinie in Richtung auf die erste Nebenecke hin. Mit dem mindestens einen Verschlusselement kann somit der durch Umfalten um die Verschlussfaltlinie gebildete Umschlag der Verpackungsvorrichtung fixiert werden, so dass er sich nicht wieder von selbst zurückschlagen kann.

Auf einfache Weise lässt sich die Verpackungsvorrichtung ausbilden und handhaben, wenn das mindestens eine Verschlusselement in Form eines Klebestreifens ausgebildet ist. Insbesondere kann es sich um einen Klebestreifen in Form eines Z-förmig auf sich zurückgefalteten Klebestreifens handeln. Derartige Z-förmige Klebestreifen sind in einer Ausgangsstellung Z-förmig gefaltet und so auf etwa ein Drittel ihrer Gesamtlänge verkürzt. Ein freies Ende kann von einem Anwender ergriffen werden, sodass die drei Lagen des Z-förmig auf sich zurückgefalteten Klebestreifens auseinandergefaltet werden können. Das andere Ende des Klebestreifens ist fest mit der zweiten Flachmaterialseitenfläche verbunden, sodass sich das Verschlusselement nicht in unerwünschter Weise von der Verpackungsvorrichtung lösen kann. Ein derart angeordnetes Verschlusselement vereinfacht die Handhabung, da ein Anwender einen solchen Klebestreifen nicht selbst beispielsweise von einer Rolle abreißen muss. Der Klebestreifen ist genau in der Länge, in der er benötigt wird, vorkonfektioniert.

Günstigerweise ist das mindestens eine Verschlusselement auf der zweiten Flachmaterialseitenfläche angeordnet oder ausgebildet. Dies ermöglicht es insbesondere, einen Flachmaterialbogen in der ungefalteten Ausgangsstellung bereitzustellen, auf dem bereits ein oder mehrere Verschlusselemente, beispielsweise Klebestreifen in Form von Z-förmigen Klebestreifen, angeordnet sind. Insbesondere können auf der zweiten Flachmaterialseitenfläche wie oben beschrieben auch ein oder mehrere Sicherungselemente angeordnet sein, beispielsweise in Form von doppelseitigen Klebestreifen, die dann beim Überführen des Flachmaterialbogens von der Ausgangsstellung in die Einführstellung lediglich noch auf einer Seite von beispielsweise einer abziehbaren Schutzfolie befreit werden müssen, um dann zwei Sicherungsflächenbereiche in der beschriebenen Weise miteinander zu verbinden.

Günstig ist es, wenn das mindestens eine Verschlusselement eine Verschlusselementlängsrichtung definiert und wenn die Verschlusselementlängsrichtung parallel oder im Wesentlichen parallel zu einer der zwei Seitenfaltlinien oder parallel oder im Wesentlichen parallel zur Hauptfaltlinie verläuft. Verläuft die Verschlusselementlängsrichtung parallel zu einer der zwei Seitenfaltlinien, kann das Verschlusselement direkt den über die Einführöffnung umgefalteten Umschlag der Verpackungsvorrichtung gegen ein Zurückfalten fixieren. Eine optionale oder zusätzliche Fixierung kann insbesondere erfolgen, wenn weitere Verschlusselemente vorgesehen sind, deren Verschlusselementlängsrichtungen parallel oder im Wesentlichen parallel zur Hauptfaltlinie verlaufen.

Vorteilhaft ist es, wenn in der der Einführstellung sich das mindestens eine Verschlusselement bis an die Löselaschenhauptfaltlinie heran oder bis an eine der beiden Seitenfaltlinien heran erstreckt und in der Verschlussstellung sich das mindestens eine Verschlusselement über die Löselaschenhauptfaltlinie vor oder über eine der beiden Seitenfaltlinien vor erstreckt. Diese vorgeschlagene Weiterbildung ermöglicht es insbesondere, einen durch Umfalten um die Verschlussfaltlinie über die Einführöffnung umgeschlagenen Umschlag der Verpackungsvorrichtung einfach und sicher zu fixieren.

Einfach und kostengünstig ausbilden lässt sich die Verpackungsvorrichtung, wenn der Flachmaterialbogen aus einem Tuch, einem Vlies, insbesondere einem Kunststoffvlies, oder einem Verpackungspapier, insbesondere einem Krepppapier, ausgebildet ist.

Für die Handhabung, insbesondere in einem Krankenhaus, ist es vorteilhaft, wenn die Verpackungsvorrichtung in Form eines Einwegprodukts ausgebildet ist. So kann eine aufwendige Aufbereitung der Verpackungsvorrichtung vermieden werden. Insbesondere lässt sich die Verpackungsvorrichtung durch Aufziehen, beispielsweise Entfalten, vom Siebkorb einfach und schnell entfernen.

Vorteilhafterweise ist die Verpackungsvorrichtung sterilisierbar, insbesondere heißdampfsterilisierbar, ausgebildet. Dies ermöglicht es insbesondere, den im Aufnahmeraum der Verpackungsvorrichtung aufgenommenen Siebkorb nach Überführen der Verpackungsvorrichtung von der Einführstellung in die Verpackungsstellung mitsamt seinem Inhalt zu sterilisieren, beispielsweise durch einen Heißdampfsterilisationsprozess.

Um eine sichere Verpackung eines Sterilbehälters gewährleisten zu können, ist es vorteilhaft, wenn die Verpackungsvorrichtung aus einem einstückigen, insbesondere monolithischen, Flachmaterialbogen ausgebildet ist.

Die eingangs gestellte Aufgabe wird ferner bei einem medizinischen Verpackungssystem der eingangs beschriebenen Art erfindungsgemäß dadurch gelöst, dass es eine Mehrzahl von medizinischen Verpackungsvorrichtungen umfasst, welche in Form einer der oben beschriebenen Ausführungsformen medizinischer Verpackungsvorrichtungen ausgebildet sind. So können insbesondere mehrere solcher Verpackungsvorrichtung bereitgestellt werden, in die ein Anwender dann bereits mit Instrumenten oder Implantaten bestückte Siebkörbe einschieben muss, um diese zu verpacken.

Günstig ist es, wenn sich mindestens zwei der Mehrzahl von medizinischen Verpackungsvorrichtungen in Form und/oder Größe und/oder durch den Werkstoff, aus dem der Flachmaterialbogen ausgebildet ist, voneinander unterscheiden. So können insbesondere unterschiedlich große vorkonfektionierte Verpackungsvorrichtungen bereitgestellt werden, um Sterilisiersiebschalen unterschiedlicher Größe und Formen in der oben beschriebenen Weise schnell und einfach verpacken zu können.

Vorteilhaft ist es, wenn das Verpackungssystem eine Umverpackung für die Mehrzahl von Verpackungsvorrichtungen umfasst. Insbesondere kann die Umverpackung in Form einer Spendervorrichtung mit einer Entnahmeöffnung ausgebildet sein, durch die hindurch die Verpackungsvorrichtungen einzeln aus der Spendervorrichtung entnehmbar sind. Beispielsweise können derartige Spendervorrichtungen in einer Aufbereitungseinheit für Medizinprodukte, früher auch als zentrale Sterilgutversorgungsabteilung bezeichnet, bereitgestellt werden. Ein Anwender entnimmt dann zum Verpacken einer Sterilisiersiebschale einer solchen Spendervorrichtung die jeweils für Form und Größe der Sterilisiersiebschale passende Verpackungsvorrichtung aus einer bereitgestellten Spendervorrichtung und schiebt dann die Sterilisiersiebschale in den Aufnahmeraum der Verpackungsvorrichtung hinein. Das bislang erforderliche aufwendige Einschlagen der Sterilisiersiebschale in einen Flachmaterialbogen ist dann nicht mehr erforderlich. Die Vorbereitung von zu sterilisierenden Gütern vor der Sterilisation kann so deutlich schneller erfolgen.

Vorzugsweise umfasst das Verpackungssystem mindestens einen Siebkorb. In den Siebkorb können insbesondere Instrumente oder Implantate eingebracht werden, die für einen medizinischen, insbesondere chirurgischen, Eingriff unter sterilen Bedingungen bereitgestellt werden sollen.

Die eingangs gestellte Aufgabe wird ferner bei einem Verfahren zum Herstellen einer medizinischen Verpackungsvorrichtung der eingangs beschriebenen Art erfindungsgemäß dadurch gelöst, dass die Verpackungsvorrichtung in der Einführstellung gegen ein vollständiges Entfalten zurück in die Ausgangsstellung gesichert wird. Wie bereits oben beschrieben, wird auf diese Weise verhindert, dass sich die Verpackungsvorrichtung in unerwünschter Weise von selbst entfalten kann. Dies erleichtert insbesondere das Einführen des Siebkorbs in den Aufnahmeraum hinein. Die Verpackungsvorrichtung kann durch das beschriebene Sichern mit einer für ihre Handhabung vorteilhaften Formstabilität bereitgestellt werden.

Günstig ist es, wenn der Flachmaterialbogen eine erste Flachmaterialseitenfläche und eine zweite Flachmaterialseitenfläche definiert, wenn die erste Flachmaterialseitenfläche und die zweite Flachmaterialseitenfläche in der Ausgangsstellung in voneinander entgegengesetzte Richtungen weisen und wenn die zwei Aufnahmeraumflachmaterialbogenflächenbereiche durch Falten des Flachmaterialbogens aus der ersten Flachmaterialseitenfläche gebildet werden. Mithin kommen also durch Falten des Flachmaterialbogens zwei Aufnahmeraumflachmaterialbogenflächenbereiche aneinander in Anlage, die beide Ausschnitte der ersten Flachmaterialseitenfläche bilden. So kann erreicht werden, dass der Aufnahmeraum lediglich durch Flächenbereiche der ersten Flachmaterialseitenfläche begrenzt wird.

Auf einfache Weise lässt sich die Verpackungsvorrichtung durch Falten des Flachmaterialbogens entlang von mindestens drei Faltlinien bilden, insbesondere entlang von drei, vier, fünf, sechs oder sieben Faltlinien. Diese Ausgestaltung ermöglicht es insbesondere, die Verpackungsvorrichtung aus einem einzigen, also insbesondere einem einstückigen, monolithischen Flachmaterialbogen auszubilden.

Die Verpackungsvorrichtung lässt sich auf einfache Weise herstellen, wenn ein Flachmaterialbogen bereitgestellt wird, welcher in der Ausgangsstellung viereckig, insbesondere rechteckig ist. Ferner kann er optional quadratisch oder im Wesentlichen quadratisch ausgebildet sein. Derartige Flachmaterialbögen können auf einfache Weise hergestellt und gefaltet werden.

Vorteilhaft ist es, wenn eine der mindestens drei Faltlinien in Form einer Hauptfaltlinie ausgebildet wird, welche zwei in der Ausgangsstellung einander gegenüberliegende Hauptecken des Flachmaterialbogens miteinander verbindet. So lässt sich beispielsweise ein quadratischer Flachmaterialbogen durch die Hauptfaltlinie halbieren.

Um die Einführöffnung in definierter Weise ausbilden zu können, ist es vorteilhaft, wenn sie sich parallel oder im Wesentlichen parallel zur Hauptfaltlinie erstreckend ausgebildet wird.

Ferner ist es günstig, wenn zwei der mindestens drei Faltlinien in Form von parallel oder im Wesentlichen parallel zueinander verlaufenden Seitenfaltlinien ausgebildet werden, welche sich quer zur Hauptfaltlinie erstrecken. So kann der Aufnahmeraum insbesondere durch die Hauptfaltlinie und die zwei Seitenfaltlinien von drei Seiten begrenzt und verschlossen werden.

Vorteilhafterweise werden die beiden Seitenfaltlinien in einem Abstand voneinander ausgebildet, welcher mindestens einem Drittel eines Abstands der Hauptecken voneinander entspricht. So kann insbesondere verhindert werden, dass die Hauptecken mehrfach umgefaltet werden müssen, wodurch sich Bereiche bilden würden, in denen sehr viele Lagen des Flachmaterialbogens übereinander liegen würden.

Einfach lässt sich die Verpackungsvorrichtung in definierter Weise ausbilden, wenn die Hauptecken durch Umfalten auf der Hauptfaltlinie oder im Wesentlichen auf der Hauptfaltlinie positioniert werden. Da die Hauptecken vor dem Umfalten um die Seitenfaltlinien bereits auf der Hauptfaltlinie liegen, denn so ist diese definiert, verlaufen die Seitenfaltlinien automatisch senkrecht zur Hauptfaltlinie.

Der Aufnahmeraum kann in definierter Weise vorgegeben werden, wenn die zwei Aufnahmeraumflachmaterialbogenflächenbereiche durch die Hauptfaltlinie begrenzt werden. Sie können ferner auch durch die zwei Seitenfaltlinien begrenzt werden.

Gemäß einer weiteren bevorzugten Ausführungsform der Erfindung kann vorgesehen sein, dass der Flachmaterialbogen in der Ausgangsstellung zwei einander gegenüberliegende Nebenecken umfasst, dass die zwei Nebenecken durch Falten des Flachmaterialbogens von der Ausgangsstellung um die Hauptfaltlinie in eine erste Faltstellung gebracht werden, in welcher sie aufeinander liegen, dass eine erste Löselasche der Verpackungsvorrichtung ausgebildet wird durch Zurückfalten einer ersten der zwei Nebenecken um eine Nebenfaltlinie aus der ersten Faltstellung in eine zweite Faltstellung und dass die Nebenfaltlinie parallel oder im Wesentlichen parallel zur Hauptfaltlinie verläuft und sich zwischen der Hauptfaltlinie und den Nebenecken in der ersten Faltstellung erstreckt. So kann insbesondere eine Löselasche ausgebildet werden, die über die Hauptfaltlinie vorsteht, so dass ein Anwender sie sicher fassen kann, um beispielsweise einen im Aufnahmeraum der Verpackungsvorrichtung aufgenommenen Sterilbehälter wieder zu entpacken.

Günstigerweise wird die erste Löselasche in der Einführstellung über die Hauptfaltlinie vorstehend ausgebildet. Wie bereits erwähnt kann so ein Anwender einen Siebkorb auf einfache und sichere Weise wieder von der Verpackungsvorrichtung befreien.

Vorteilhaft ist es, wenn die Nebenfaltlinie ausgebildet wird mit einem Abstand von der Hauptfaltlinie, welcher kleiner ist als von den Nebenecken in der ersten Faltstellung. Diese Vorgabe für den Abstand kann insbesondere sicherstellen, dass die erste Löselasche in der Einführstellung über die Hauptfaltlinie vorsteht.

Günstig ist es, wenn die Nebenfaltlinie die Einführöffnung begrenzend ausgebildet wird. So kann ein Anwender insbesondere stets direkt erkennen, wo die Einführöffnung ist. Dies erleichtert ihm das Einführen eines Siebkorbs in den Aufnahmeraum der Verpackungsvorrichtung.

Vorteilhaft ist es, wenn eine erste der zwei Hauptecken von der zweiten Faltstellung um eine erste der zwei Seitenfaltlinien in Richtung auf eine zweite der zwei Hauptecken hin in eine dritte Faltstellung umgefaltet wird und wenn die zweite Hauptecke von der dritten Faltstellung in eine vierte Faltstellung um eine zweite der zwei Seitenfaltlinien in Richtung auf die erste Seitenfaltlinie hin umgefaltet wird. Durch diese beiden Faltungen kann der Aufnahmeraum in definierter Weise von drei Seiten begrenzt werden. Insbesondere können die umgefalteten Hauptecken mit derjenigen Fläche, auf der sie nach dem Umfalten zu liegen kommen, mit einem Sicherungselement gesichert werden, insbesondere kraft- und/oder stoffschlüssig.

Günstigerweise wird die Verpackungsvorrichtung derart ausgebildet, dass die vierte Faltstellung die Einführstellung definiert. Es werden also dann nur vier Faltungen benötigt, um die Verpackungsvorrichtung so vorzukonfektionieren, dass sie die Einführstellung einnimmt, in der ein Siebkorb einfach und sicher in den Aufnahmeraum eingebracht werden kann.

Vorteilhaft ist es, wenn eine zweite Löselasche der Verpackungsvorrichtung ausgebildet wird durch Umfalten einer zweiten der zwei Nebenecken, welche in der Einführstellung von der Einführöffnung weg weist, von der vierten Faltstellung in eine fünfte Faltstellung um eine Löselaschenhauptfaltlinie in Richtung auf die Hauptfaltlinie hin und durch Zurückfalten der zweiten Nebenecke von der fünften Faltstellung in eine sechste Faltstellung um eine Löselaschennebenfaltlinie in einer Richtung von der Hauptfaltlinie weg. Diese Ausgestaltung ermöglicht es einem Anwender insbesondere, die Verpackungsvorrichtung nicht nur an der erste Löselasche zu fassen, sondern auch an der zweiten Löselasche, um sie vom Siebkorb zu entfernen.

Günstig ist es, wenn die Löselaschenhauptfaltlinie ausgebildet wird mit einem Abstand von der Hauptfaltlinie, welcher größer ist als von den Nebenecken in der ersten Faltstellung, insbesondere mehr als doppelt so groß. Durch diese Abstandsvorgabe kann insbesondere sichergestellt werden, dass die zweite Nebenecke über die Löselaschenhauptfaltlinie vorsteht und so von einem Anwender in definierter Weise und sicher erfasst werden kann.

Vorteilhaft ist es, wenn die zweite Löselasche ausgebildet wird derart, dass ein Abstand der Löselaschennebenfaltlinie von der Löselaschenhauptfaltlinie größer ist als ein Abstand von der zweiten Nebenecke, insbesondere mindestens 30% größer. Durch diese Abstandsvorgabe kann insbesondere erreicht werden, dass die zweite Löselasche eine hinreichende Größe aufweist, damit ein Anwender sie zum Entfernen der Verpackungsvorrichtung von einem Siebkorb sicher ergreifen kann.

Auf einfache Weise lässt sich das Verfahren durchführen, wenn die Löselaschenhauptfaltlinie und die Löselaschennebenfaltlinie parallel oder im Wesentlichen parallel zueinander verlaufend ausgebildet werden. Insbesondere können sie parallel zur Hauptfaltlinie ausgebildet werden.

Vorzugsweise wird die Verpackungsvorrichtung derart ausgebildet, dass die Einführöffnung in der sechsten Faltstellung offen ist. Dies hat insbesondere den Vorteil, dass ein Anwender die zweite Löselasche ebenfalls schon vorkonfektioniert zur Verfügung gestellt bekommt und diese Löselasche dann nicht selbst durch die beschriebenen Faltungen um die Löselaschenhauptfaltlinie und die Löselaschennebenfaltlinie ausbilden muss.

Um zu verhindern, dass sich die Verpackungsvorrichtung ausgehend von der Einführstellung wieder in unerwünschter Weise in die Ausgangsstellung des Flachmaterialbogens zurück entfalten kann, ist es vorteilhaft, wenn die Verpackungsvorrichtung mit mindestens einem Sicherungselement in der Einführstellung gesichert wird. Beispielsweise können so durch Falten aufeinanderliegende Bereiche des Flachmaterialbogens aneinander fixiert werden, um der Verpackungsvorrichtung eine für eine gute Handhabbarkeit vorteilhafte Formstabilität zu verleihen.

Günstig ist es, wenn die Verpackungsvorrichtung mindestens einen ersten Sicherungsflächenbereich und mindestens einen zweiten Sicherungsflächenbereich definiert, wenn der erste Sicherungsflächenbereich und der zweite Sicherungsflächenbereich von den zwei in der Einführstellung aneinander anliegenden Aufnahmeraumflachmaterialbogenflächenbereichen verschieden sind, wenn der erste Sicherungsflächenbereich und der zweite Sicherungsflächenbereich in der Einführstellung aneinander anliegen und wenn mit dem mindestens einen Sicherungselement der mindestens eine erste Sicherungsflächenbereich und der mindestens eine zweite Sicherungsflächenbereich kraft- und/oder stoffschlüssig miteinander verbunden werden, insbesondere durch Kleben und/oder Schweißen. Die beiden Sicherungsflächenbereiche können insbesondere aneinander anliegende Flächenbereiche des Flachmaterialbogens definieren, die nach Falten desselben um eine der Faltlinien aneinander anliegen. Ein Zurückfalten kann also dann verhindert werden, wenn diese miteinander verbunden werden, beispielsweise durch Kleben und/oder Schweißen. Insbesondere kann hier ein Klebstoff eingesetzt werden oder ein doppelseitiger Klebestreifen. Ist der Flachmaterialbogen aus einem Kunststoff ausgebildet, können die Sicherungsflächenbereiche insbesondere auch durch Ultraschallschweißen miteinander stoffschlüssig verbunden werden.

Vorteilhaft ist es, wenn die Verpackungsvorrichtung derart ausgebildet wird, dass der mindestens eine erste Sicherungsflächenbereich und der zugeordnete mindestens eine zweite Sicherungsflächenbereich von derselben Flachmaterialseitenfläche oder von unterschiedlichen Flachmaterialseitenflächen umfasst sind. Insbesondere ist es möglich, dass nur Sicherungsflächenbereiche, die von der zweiten Flachmaterialseitenfläche umfasst sind, also diejenige Flachmaterialseitenfläche, die nicht den Aufnahmeraum begrenzt, miteinander verbunden werden. So kann insbesondere vermieden werden, dass die beiden Aufnahmeraumflachmaterialbogenflächenbereiche miteinander verbunden werden, wodurch der Aufnahmeraum verkleinert oder ganz zerstört werden könnte.

Auf einfache Weise lässt sich die Verpackungsvorrichtung ausbilden, wenn das mindestens eine Sicherungselement durch einen Schweißpunkt, durch einen Klebstoff oder durch ein Klebeelement ausgebildet wird. Insbesondere kann das Klebeelement zwei voneinander weg weisende Klebeflächen aufweisen und derart angebracht werden, insbesondere in der Ausgangsstellung des Flachmaterialbogens, dass in der Einführstellung die eine der zwei Klebeflächen am mindestens einen ersten Sicherungsflächenbereich anliegt und dass die andere der zwei Klebeflächen am mindestens einen zweiten Sicherungsflächenbereich anliegt. Insbesondere dann, wenn das Klebeelement, welches als doppelseitiger Klebestreifen ausgebildet ist, bereits auf den Flachmaterialbogen in der Ausgangsstellung an einem der Sicherungsflächenbereiche angebracht ist, muss beim Herstellen der Verpackungsvorrichtung beispielsweise lediglich noch eine Schutzfolie am Klebeelement abgezogen werden, um dieses mit einem weiteren Seitenflächenbereich zu verbinden und so die Verpackungsvorrichtung in der Einführstellung zu sichern.

Um der Verpackungsvorrichtung in der Einführstellung eine hohe Formstabilität zu verleihen, ist es vorteilhaft, wenn das mindestens eine Sicherungselement in einem Bereich der Verpackungsvorrichtung positioniert wird, in welchem in der Einführstellung mindestens zwei durch Umfalten des Flachmaterialbogens ausgebildete Lagen aufeinanderliegen.

Gemäß einer weiteren bevorzugten Ausführungsform der Erfindung kann vorgesehen sein, dass die Verpackungsvorrichtung mit mindestens einem Verschlusselement ausgebildet wird zum Verschließen der Verpackungsvorrichtung in einer Verpackungsstellung, in welcher die Einführöffnung verschlossen ist durch Umfalten der zweiten Nebenecke um eine Verschlussfaltlinie in Richtung auf die erste Nebenecke hin. Ein solches Verschlusselement vorzusehen vereinfacht einem Anwender das Verpacken eines Siebkorbs, da er ein solches Verschlusselement nicht separat, beispielsweise von einer Klebestreifenrolle, abziehen muss. Das Verschlusselement ist für ihn bereits dort positioniert, wo er es benötigt, und idealerweise auch entsprechend dimensioniert.

Für die Handhabung der Verpackungsvorrichtung ist es günstig, wenn das mindestens eine Verschlusselement in Form eines Klebestreifens ausgebildet wird. Insbesondere kann es in Form eines Z-förmig auf sich zurückgefalteten Klebestreifens ausgebildet werden.

Vorzugsweise wird das mindestens eine Verschlusselement auf der zweiten Flachmaterialseitenfläche angeordnet oder ausgebildet. Insbesondere kann es dort bereits in der Ausgangsstellung des Flachmaterialbogens angeordnet oder ausgebildet werden. Insbesondere ist es möglich, alle Verschlusselemente und alle Sicherungselemente bereits in der Ausgangsstellung auf dem Flachmaterialbogen anzuordnen oder zu befestigen. Dies vereinfacht die Herstellung der Verpackungsvorrichtung, da bereits der Flachmaterialbogen in der beschriebenen Weise mit Sicherungs- und Verschlusselementen vorkonfektioniert werden kann. Er muss dann von der Ausgangsstellung lediglich durch mehrere Faltungen in die Einführstellung überführt werden.

Günstigerweise definiert das mindestens eine Verschlusselement eine Verschlusselementlängsrichtung und wird derart auf der Verpackungsvorrichtung angeordnet, dass die Verschlusselementlängsrichtung parallel oder im Wesentlichen parallel zu einer der zwei Seitenfaltlinien oder parallel oder im Wesentlichen parallel zur Hauptfaltlinie verläuft. So kann insbesondere ein über die Einführöffnung umgefalteter Umschlag mit der zweiten Nebenecke schnell und sicher fixiert werden, nämlich beispielsweise in einer Richtung quer, insbesondere senkrecht zur Einführöffnung, wenn diese parallel zur Hauptfaltlinie verläuft, oder auch quer zu dieser Richtung.

Günstig ist es, wenn das mindestens eine Verschlusselement derart angeordnet wird, dass es sich in der Einführstellung bis an die Löselaschenhauptfaltlinie heran oder bis an eine der beiden Seitenfaltlinien heran erstreckt und sich in der Verschlussstellung über die Löselaschenhauptfaltlinie vor oder über eine der beiden Seitenfaltlinien vor erstreckt. Die Verschlussstellung wird insbesondere definiert als diejenige Stellung, in welcher die Einführöffnung verschlossen ist. Das Verschlusselement muss dann lediglich über die Löselaschenhauptfaltlinie hinweg oder über die jeweilige Seitenfaltlinie hinweg geführt werden, um den die Einführöffnung verschließenden Umschlag des Flachmaterialbogens seitlich zu fixieren.

Auf einfache Weise herstellen lässt sich die Verpackungsvorrichtung, wenn der Flachmaterialbogen aus einem Tuch, einem Vlies, insbesondere einem Kunststoffvlies, oder einem Verpackungspapier, insbesondere einem Krepppapier, ausgebildet wird.

In definierter Weise lassen sich Siebkörbe steril verpacken, wenn die Verpackungsvorrichtung in Form eines Einwegprodukts ausgebildet wird. Insbesondere kann die Verpackungsvorrichtung aus einem sterilen Flachmaterialbogen ausgebildet werden.

Damit eine Sterilisiersiebschale, die mit einer Verpackungsvorrichtung verpackt ist, sterilisiert werden kann, ist es günstig, wenn die Verpackungsvorrichtung sterilisierbar, insbesondere heißdampfsterilisierbar, ausgebildet wird. Hierfür können entsprechende Werkstoffe gewählt werden, aus denen der Flachmaterialbogen ausgebildet werden kann.

Um insbesondere unerwünschte Öffnung der Verpackungsvorrichtung zu vermeiden, ist es vorteilhaft, wenn die Verpackungsvorrichtung aus einem einstückigen, insbesondere monolithischen, Flachmaterialbogen ausgebildet wird.

Die eingangs gestellte Aufgabe wird bei einem Verfahren zum sterilen Verpacken eines Siebkorbs der eingangs beschriebenen Art erfindungsgemäß dadurch gelöst, dass eine der oben beschriebenen medizinischen Verpackungsvorrichtungen bereitgestellt wird, dass der Siebkorb durch die Einführöffnung in den Aufnahmeraum eingeführt wird und dass die Verpackungsvorrichtung von der Einführstellung in eine Verschlussstellung, in welcher die Einführöffnung verschlossen ist, überführt wird durch Umschlagen eines freien Endes der Verpackungsvorrichtung über die Einführöffnung.

In der oben bereits eingehend beschriebenen Weise ist es so möglich, einen Siebkorb signifikant schneller zu verpacken. Der Flachmaterialbogen muss insbesondere nicht mehr von einem Anwender um den Siebkorb aufwendig herum gefaltet werden, was einen hohen Zeit- und damit auch Kostenaufwand verursacht. Der Siebkorb muss lediglich durch die Einführöffnung in den Aufnahmeraum eingeschoben werden. Durch Umschlagen eines freien Endes der Verpackungsvorrichtung über die Einführöffnung wird diese verschlossen. Der Siebkorb ist dann optimal verpackt. Dieses Verfahren können insbesondere auch Anwender durchführen, die nicht aufwendig geschult sind.

Vorzugsweise wird das umgeschlagene freie Ende in der Verschlussstellung mit mindestens einem Verschlusselement fixiert. Insbesondere können zwei, drei, vier oder auch noch mehrere Verschlusselemente vorgesehen werden, um das umgeschlagene freie Ende in der Verschlussstellung zu fixieren. Ein Siebkorb ist so sicher verpackt. Die Verpackungsvorrichtung kann sich nicht in unbeabsichtigter Weise vom Siebkorb lösen.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Darstellung eines Ausführungsbeispiels eines Flachmaterialbogens zur Ausbildung einer Verpackungsvorrichtung in einer ungefalteten Ausgangsstellung;
- Figur 2:: eine schematische Darstellung des Flachmaterialbogens aus Figur 1 in einer ersten Faltstellung;
- Figur 3:: eine schematische perspektivische Ansicht des Flachmaterialbogens beim Überführen von der ersten Faltstellung in eine zweite Faltstellung;
- Figur 4:: eine schematische perspektivische Anordnung des Flachmaterialbogens in der zweiten Faltstellung;
- Figur 5:: eine schematische perspektivische Ansicht des Flachmaterialbogens aus Figur 4 beim Überführen von der zweiten Faltstellung in eine dritte Faltstellung;
- Figur 6:: eine schematische perspektivische Ansicht des Flachmaterialbogens aus Figur 5 beim Überführen von der dritten Faltstellung in eine vierte Faltstellung;
- Figur 7:: eine schematische Darstellung des Flachmaterialbogens in der vierten Faltstellung, die eine Einführstellung definiert;
- Figur 8:: eine schematische Darstellung der Anordnung aus Figur 7 beim Einführen eines Siebkorbs durch eine Einführöffnung der Verpackungsvorrichtung in einen Aufnahmeraum derselben;
- Figur 9:: eine schematische Darstellung der Anordnung aus Figur 8 beim Überführen von der vierten Faltstellung in eine fünfte Faltstellung sowie in eine sechste Faltstellung;
- Figur 10:: eine schematische Darstellung der Anordnung aus Figur 9 beim Überführen von der sechsten Faltstellung in eine Verschlussstellung;
- Figur 11:: eine schematische Darstellung der Anordnung aus Figur 10 in der Verschlussstellung vor dem Aktivieren von Verschlusselementen;
- Figur 12:: eine schematische perspektivische Gesamtansicht der Anordnung aus Figur 11 mit aktivierten Verschlusselementen;
- Figur 13:: eine schematische perspektivische vergrößerte Ansicht des Bereichs A aus Figur 6;
- Figur 14:: eine schematische vergrößerte Ansicht des Bereichs B aus Figur 11;
- Figur 15:: eine schematische Darstellung einer Draufsicht auf eine zweite Flachmaterialseitenfläche eines Flachmaterialbogens; und
- Figur 16:: eine schematische Darstellung eines Ausführungsbeispiels eines medizinischen Verpackungssystems.

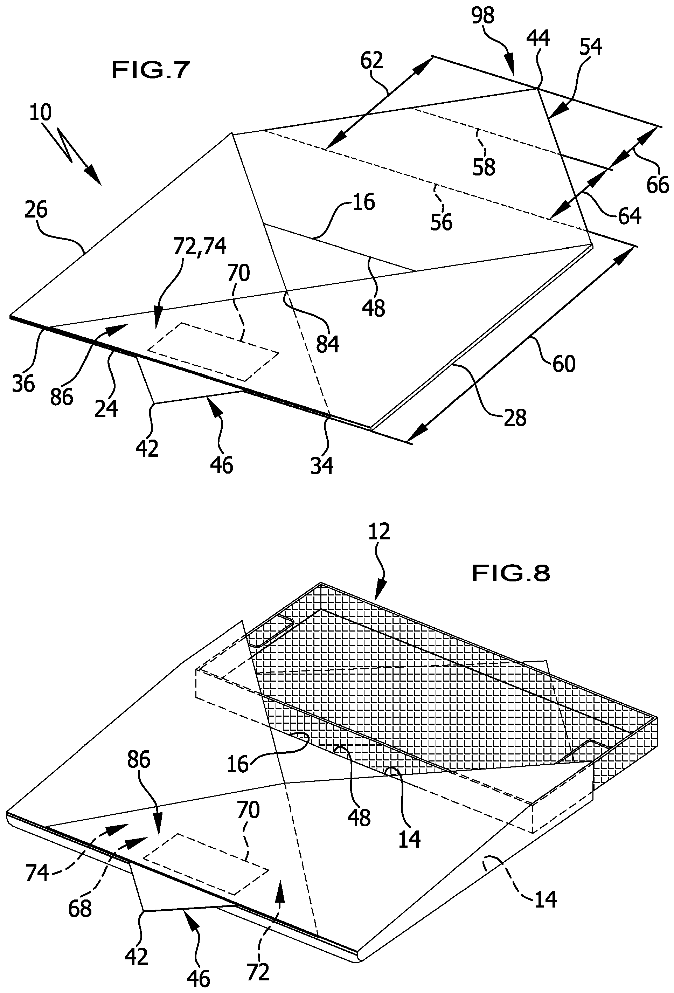

In Figur 7 ist ein Ausführungsbeispiel einer medizinischen Verpackungsvorrichtung schematisch dargestellt und insgesamt mit dem Bezugszeichen 10 bezeichnet. Sie dient zum sterilen Verpacken eines Siebkorbs 12, welcher schematisch in Figur 8 dargestellt ist. In Figur 8 sind die normalerweise in den Siebkorb 12 eingebrachten Instrumente oder Implantate der Übersichtlichkeit wegen nicht dargestellt.

Die Verpackungsvorrichtung 10 definiert einen Aufnahmeraum 14 zum Aufnehmen des Siebkorbs 12 in einer Verpackungsstellung.

Die Verpackungsvorrichtung 10 definiert ferner eine Einführöffnung 16, durch die hindurch der Siebkorb 12 in den Aufnahmeraum 14 hineingebracht werden kann.

In der in Figur 8 dargestellten Einführstellung der Verpackungsvorrichtung 10 ist die Einführöffnung 16 offen.

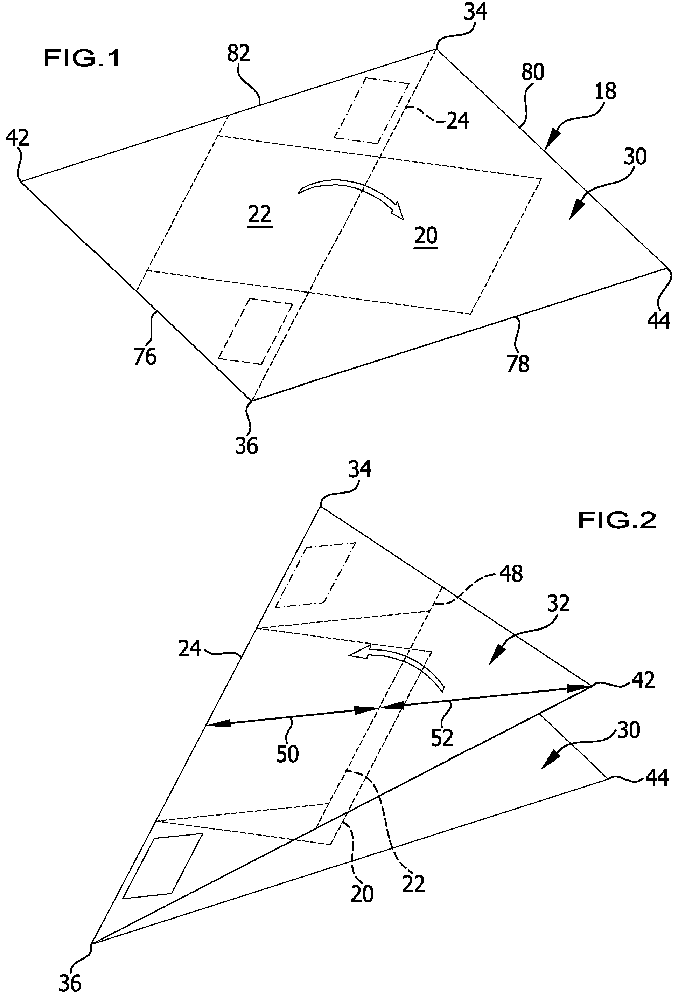

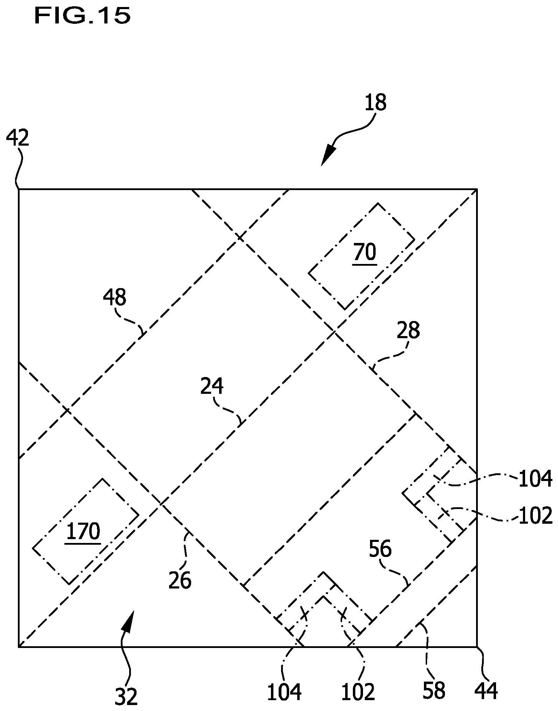

Die Verpackungsvorrichtung 10 ist aus einem Flachmaterialbogen 18 ausgebildet. Ein solcher Flachmaterialbogen 18 ist schematisch in Figur 1 dargestellt. Auch die Figur 15 zeigt schematisch einen Flachmaterialbogen 18. Die Verpackungsvorrichtung 10 wird aus dem Flachmaterialbogen 18 durch mehrfaches Falten gebildet, wie nachfolgend noch eingehend erläutert wird.

In der Einführstellung weist der Aufnahmeraum 14 zwei aneinander anliegende Aufnahmeraumflachmaterialbogenflächenbereiche, nämlich einen ersten Aufnahmeraumflachmaterialbogenflächenbereich 20 und einen zweiten Aufnahmeraumflachmaterialbogenflächenbereich 22 identischer Größe, die schematisch in den Figuren 1 und 2 eingezeichnet sind.

Die beiden Aufnahmeraumflachmaterialbogenflächenbereiche 20 und 22 sind durch drei Faltlinien des Flachmaterialbogens 18 begrenzt, nämlich eine Hauptfaltlinie 24 und zwei Seitenfaltlinien, nämlich eine erste Seitenfaltlinie 26 und eine zweite Seitenfaltlinie 28. Die Hauptfaltlinie 24 sowie die Seitenfaltlinien 26 und 28 begrenzen den Aufnahmeraum 14 derart, dass er bis auf die Einführöffnung 16 allseitig geschlossen ist.

Der Flachmaterialbogen 18 definiert eine erste Flachmaterialseitenfläche 30 und eine zweite Flachmaterialseitenfläche 32, die in der Ausgangsstellung des ungefalteten Flachmaterialbogens 18 in voneinander entgegengesetzte Richtungen weisen. Wie schematisch in Figur 1 gestrichelt eingezeichnet, umfasst die erste Flachmaterialseitenfläche 30 die zwei Aufnahmeraumflachmaterialbogenflächenbereiche 20 und 22. Figur 15 zeigt eine Draufsicht auf die Flachmaterialseitenfläche 32.

Der Flachmaterialbogen 18 ist der Ausgangsstellung viereckig, nämlich wie beim Ausführungsbeispiel in Figur 1 schematisch dargestellt, quadratisch. Eine der drei genannten Faltlinien ist in Form der Hauptfaltlinie 24 ausgebildet, welche zwei in der Ausgangsstellung einander gegenüberliegende Hauptecken 34 und 36 des Flachmaterialbogens 18 miteinander verbindet, nämlich eine erste Hauptecke 34 und eine zweite Hauptecke 36.

Die Haupfaltlinie 24 erstreckt sich parallel zur Einführöffnung 16. Die Seitenfaltlinien 28 und 30 verlaufen parallel zueinander und erstrecken sich quer zur Hauptfaltlinie 24, nämlich senkrecht zu dieser. Ein Abstand 38 der beiden Seitenfaltlinien 28 und 30 voneinander entspricht mindestens einem Drittel eines Abstands 40 der beiden Hauptecken 34 und 36 voneinander.

Wie schematisch in Figur 7 dargestellt, sind die Hauptecken 34 und 36 in der Einführstellung auf der Hauptfaltlinie 24 positioniert.

Die Hauptfaltlinie 24 begrenzt ferner die zwei Aufnahmeraumflachmaterialbogenflächenbereiche 20 und 22, wie dies schematisch in Figur 1 dargestellt ist.

Der Flachmaterialbogen 18 definiert in der Ausgangsstellung zwei einander gegenüberliegende Nebenecken, nämlich eine erste Nebenecke 42 und eine zweite Nebenecke 44.

Die zwei Nebenecken 42 und 44 liegen in einer ersten Faltstellung, wie sie schematisch in Figur 2 dargestellt ist, in welcher der Flachmaterialbogen 18 von der Ausgangsstellung um die Hauptfaltlinie 24 gefaltet ist, aufeinander.

Die Verpackungsvorrichtung 10 umfasst eine erste Löselasche 46, welche durch Zurückfalten der ersten Nebenecke 42 aus der ersten Faltstellung in eine zweite Faltstellung um eine Nebenfaltlinie 48 ausgebildet ist. Die Nebenfaltlinie 48 verläuft parallel zur Hauptfaltlinie 24. Sie erstreckt sich zwischen der Hauptfaltlinie 24 und den Nebenecken 42 und 44 in der ersten Faltstellung.

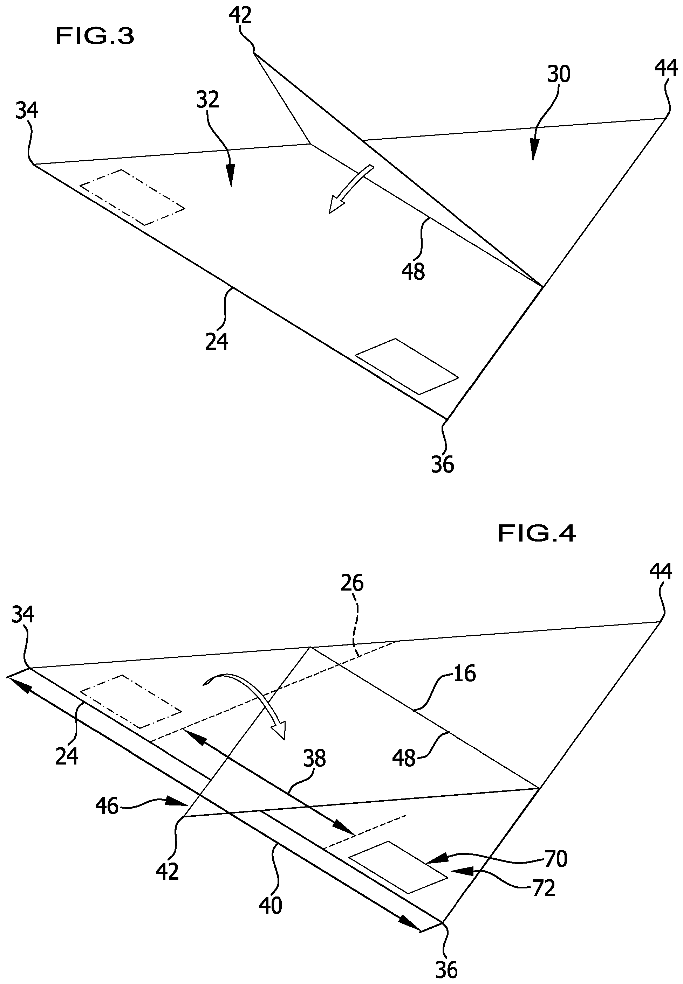

Wie insbesondere gut in den Figuren 4 bis 12 zu erkennen, steht die erste Löselasche 46, die in Form eines dreieckigen Zipfels mit der ersten Nebenecke 42 ausgebildet ist, in der Einführstellung über die Hauptfaltlinie 24 vor.

Ein Abstand 50 der Nebenfaltlinie 48 von der Hauptfaltlinie 24 ist kleiner als ein Abstand 52 von den Nebenecken 42 beziehungsweise 44 in der ersten Faltstellung, wie dies schematisch in Figur 2 dargestellt ist.

Wie insbesondere in den Figuren 7 und 8 gut zu erkennen, begrenzt die Nebenfaltlinie 48 die Einführöffnung 16.

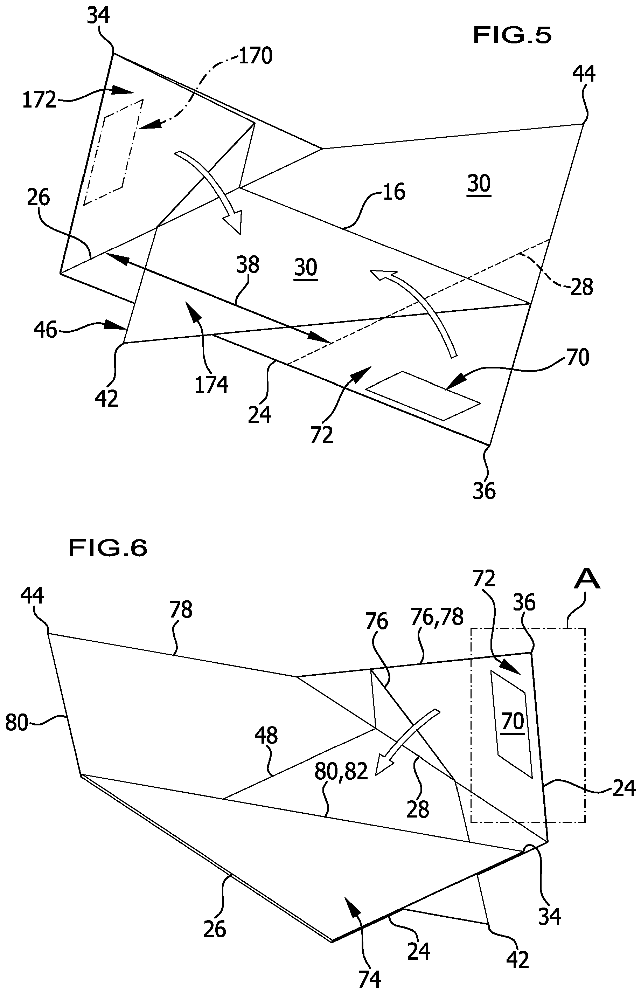

Die zweite Faltstellung ist schematisch in Figur 4 dargestellt. Ausgehend von der zweiten Faltstellung wird die erste Hauptecke 34 um die erste Seitenfaltlinie 26 in Richtung auf die zweite Hauptecke 36 hin in eine dritte Faltstellung umgefaltet, wie sie schematisch in Figur 6 dargestellt ist. Der Faltvorgang ist in Figur 5 schematisch dargestellt.

Die zweite Hauptecke 36 wird von der dritten Faltstellung in eine in Figur 7 schematisch dargestellte vierte Faltstellung überführt, und zwar durch Umfalten um die zweite Seitenfaltlinie 28 in Richtung auf die erste Seitenfaltlinie 26 hin. Wie bereits oben erwähnt, definiert die vierte Faltstellung die Einführstellung, in welcher der Siebkorb 12 durch die Einführöffnung 16 in den Aufnahmeraum 14 eingebracht werden kann.

Die Verpackungsvorrichtung 10 umfasst ferner eine zweite Löselasche 54. Die zweite Löselasche 54 ist ausgebildet durch Umfalten der zweiten Nebenecke 44, welche in der Einführstellung von der Einführöffnung 16 weg weist, von der vierten Faltstellung in eine fünfte Faltstellung um eine Löselaschenhauptfaltlinie 56 in Richtung auf die Hauptfaltlinie 24 hin. Ferner wird die zweite Nebenecke 44 von der fünften Faltstellung in eine sechste Faltstellung um eine Löselaschennebenfaltlinie 58 in einer Richtung von der Hauptfaltlinie 24 weg zurückgefaltet.

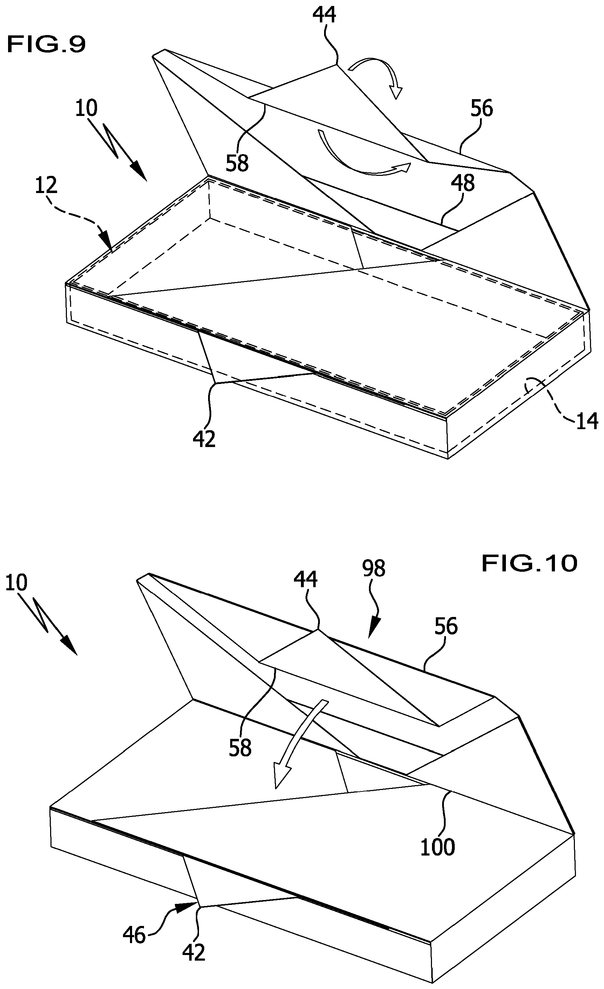

Wie insbesondere in den Figuren 9 und 10 zu erkennen, ist die Einführöffnung 16 auch in der sechsten Faltstellung offen, also wenn die zweite Löselasche 54 ausgebildet ist. Mithin definiert also auch die sechste Faltstellung eine Einführstellung der Verpackungsvorrichtung 10.

Ein Abstand 60 der Löselaschenhauptfaltlinie 56 von der Hauptfaltlinie 24 ist, wie schematisch in Figur 7 dargestellt, größer als ein Abstand 62 von den Nebenecken 42 beziehungsweise 44 in der ersten Faltstellung. Bei dem in den Figuren dargestellten Ausführungsbeispiel ist der Abstand 60 mehr als doppelt so groß wie der Abstand 62.

Ferner ist ein Abstand 64 der Löselaschennebenfaltlinie 58 von der Löselaschenhauptfaltlinie 56 größer als ein Abstand 66 von der zweiten Nebenecke 44.

Die Verpackungsvorrichtung 10 umfasst ferner eine Entfaltungssicherungseinrichtung 68 zum Sichern der Verpackungseinrichtung 10, insbesondere einer Formstabilität derselben, in der Einführstellung, und zwar gegen ein vollständiges Entfalten zurück in die Ausgangsstellung.

Die Löselaschenhauptfaltlinie 56 und die Löselaschennebenfaltlinie 58 verlaufen parallel zueinander und parallel zur Hauptfaltlinie 24.

Die Entfaltungssicherungseinrichtung 68 umfasst ein erstes Sicherungselement 70 zum Sichern der Verpackungsvorrichtung 10 in der Einführstellung.

Die Verpackungsvorrichtung 10 umfasst mindestens einen ersten Sicherungsflächenbereich 72 und einen zweiten Sicherungsflächenbereich 74. Es handelt sich dabei um Flächenbereiche des Flachmaterialbogens 18, die von den Aufnahmeraumflachmaterialbogenflächenbereichen 20 und 22 verschieden sind.

Der erste Sicherungsflächenbereich 72 und der zweite Sicherungsflächenbereich 74 liegen in der Einführstellung aneinander an. Das Sicherungselement 70 verbindet die beiden Sicherungsflächenbereiche 72 und 74 kraft- und/oder stoffschlüssig miteinander.

Bei dem in den Figuren dargestellten Ausführungsbeispiel umfasst die zweite Flachmaterialseitenfläche 32 beide Sicherungsflächenbereiche 72 und 74.

Der erste Sicherungsflächenbereich 72 wird begrenzt durch die Hauptfaltlinie 24, die zweite Seitenfaltlinie 28, eine sich zwischen der zweiten Hauptecke 36 und der ersten Nebenecke 42 erstreckende Seitenkante 76 sowie eine sich zwischen der zweiten Hauptecke 36 und der zweiten Nebenecke 44 erstreckende Seitenkante 78.

Der zweite Sicherungsflächenbereich 74 ist begrenzt durch die Hauptfaltlinie 24, eine sich zwischen der zweiten Nebenecke 44 und der ersten Hauptecke 34 erstreckende Seitenkante 80 sowie einer sich zwischen der ersten Hauptecke 34 und der ersten Nebenecke 42 erstreckenden Seitenkante 82. Ferner ist der zweite Sicherungsflächenbereich 74 begrenzt durch die erste Seitenfaltlinie 28. Dies ist schematisch in Figur 6 eingezeichnet.

Die zwei Sicherungsflächenbereiche 72 und 74 überlappen sich in einer dreieckigen Fläche 86, welche schematisch in Figur 7 eingezeichnet ist. Eine Basis dieser dreieckigen Fläche 86 ist gebildet durch die Hauptfaltlinie 24. Dieser gegenüberliegend ist eine Spitze 84 der Fläche, die in Richtung auf die Nebenfaltlinie 48 hin weist. Das Sicherungselement 70 ist zwischen den Sicherungsflächenbereichen 72 und 74 im Bereich dieser dreieckigen Fläche 86 angeordnet. Mit diesem Sicherungselement 70 ist die Verpackungsvorrichtung 10 gegen Entfalten aus der Einführstellung zurück in die Ausgangsstellung gesichert. Das Sicherungselement 70 ist derart ausgebildet, dass eine Stärke der Verbindung derart gewählt ist, dass sie beim Entfernen der Verpackungsvorrichtung 10 vom Siebkorb 12, auch als aseptische Präsentation des Siebkorbs bezeichnet, wieder gelöst werden kann, ohne den Flachmaterialbogen 18 zu zerstören.

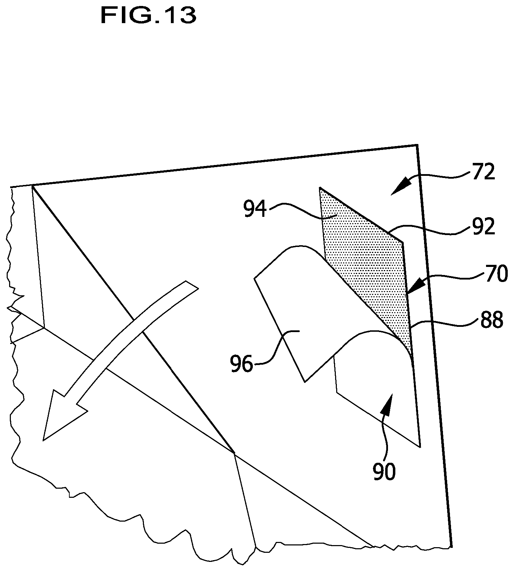

Das Sicherungselement 70 ist, wie schematisch in Figur 13 dargestellt, in Form eines doppelseitigen Klebestreifens 88 ausgebildet, welcher ein Klebeelement 90 bildet. Der Klebestreifen 88 weist eine erste Klebefläche 92 und eine zweite Klebefläche 94 auf, die in entgegensetzte Richtungen weisend ausgebildet sind.

Die erste Klebefläche 92 liegt am ersten Sicherungsflächenbereich 72 an. Die zweite Klebefläche 94 ist zunächst durch eine Schutzfolie 96 geschützt. Wird die Schutzfolie 96, wie schematisch in Figur 13 dargestellt, abgezogen, wird die zweite Klebefläche 94 freigelegt und kann dann am zweiten Sicherungsflächenbereich 74 in Anlage gebracht und mit diesem verbunden werden.

Das Sicherungselement 70 ist in einem Bereich der Verpackungsvorrichtung 10 positioniert, in welchem in der Einführstellung mindestens zwei durch Umfalten des Flachmaterialbogens 18 ausgebildete Lagen aufeinanderliegen, nämlich sieben Lagen. Bei einem alternativen Ausführungsbeispiel ist das Sicherungselement 70 in Form eines Schweißpunkts oder einen Klebstoffpunkts ausgebildet.

Ferner kann in analoger Weise wie das Sicherungselement 70 am ersten Sicherungselementflächenbereich 72 nahe der ersten Hauptecke 34, wie schematisch in Figur 5 dargestellt, ein weiteres Sicherungselement 170 angeordnet werden. Dies ist dann an einem ersten Sicherungsflächenbereich 172 angeordnet, welcher mit einem zweiten Sicherungsflächenbereich 174 zusammenwirkt, welcher durch einen Flächenbereich der ersten Flachmaterialseitenfläche 30 definiert ist. Mit dem zweiten Sicherungselement 170 lässt sich in analoger Weise wie die zweite Hauptecke 36 auch die erste Hauptecke 34 sichern, und zwar an dem um die Nebenfaltlinie 48 umgefalteten Umschlag, welcher mit der ersten Nebenecke 42 teilweise über die Hauptfaltlinie 24 vorsteht. Umfasst die Entfaltungssicherungseinrichtung 68 also nicht nur das Sicherungselement 70, sondern auch noch ein weiteres Sicherungselement 170 wie beschrieben, kann eine Formstabilität der Verpackungsvorrichtung 10 weiter verbessert werden.

Werden die Sicherungselemente 70 und 170 in der beschriebenen Weise angeordnet, liegen sie in der Einführstellung übereinander.

Ist der Siebkorb 12, wie beschrieben durch die Einführöffnung 16 in den Aufnahmeraum 14 eingeführt, kann die Verpackungsvorrichtung 10 von der Einführstellung in eine Einführstellung in eine Verschlussstellung überführt werden. In der Verschlussstellung ist die Einführöffnung 16 verschlossen, und zwar durch Umschlagen eines freien Endes 98 der Verpackungsvorrichtung 10 über die Einführöffnung 16, wie dies schematisch in Figur 10 dargestellt ist. Das freie Ende 98 umfasst insbesondere den Abschnitt der Verpackungsvorrichtung 10 mit der zweiten Löselasche 54. Das Umschlagen des freien Endes 98 erfolgt um eine Verschlussfaltlinie 100.

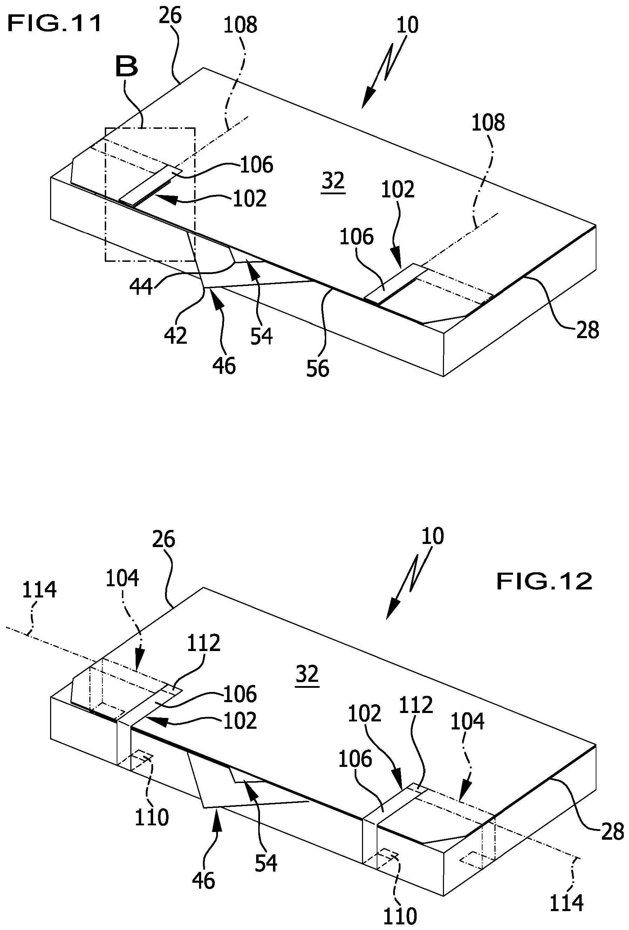

Die Figuren 11 und 12 zeigen die Verschlussstellung der Verpackungsvorrichtung 10.

Damit sich das freie Ende 98 nicht in unbeabsichtigter Weise löst, ist es in der Verschlussstellung mit mindestens einem Verschlusselement 102, bei dem in den Figuren dargestellten Ausführungsbeispiel sind es zwei Verschlusselemente 102, fixiert.

Die Verschlusselemente 102 sind in Form eines Klebestreifens 106 ausgebildet. Die Verschlusselemente 102 definieren jeweils eine Verschlusselementlängsrichtung 108, die parallel zu den zwei Seitenfaltlinien 26 und 28 verläuft.

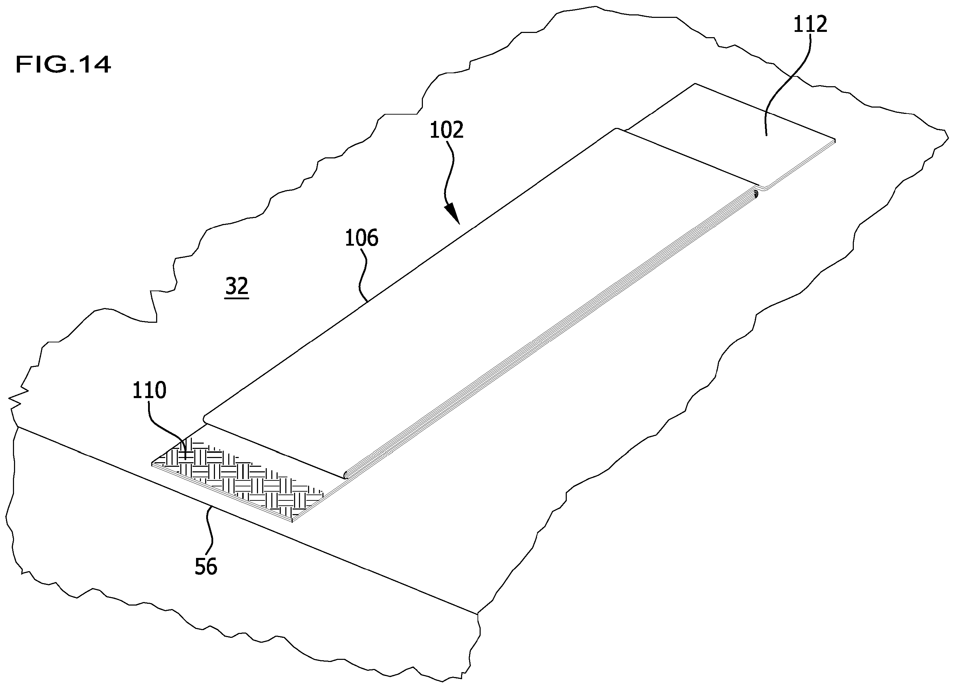

Die Verschlusselemente 102 sind in Form Z-förmiger, auf sich zurückgefalteter Klebestreifen 106 ausgebildet, wie dies schematisch vergrößert in Figur 14 dargestellt ist. Die Verschlusselemente sind auf der zweiten Flachmaterialseitenfläche 32 angeordnet.

In der Einführstellung, also bevor das freie Ende 98 mit den Verschlusselementen 102 fixiert ist, erstrecken sich die Verschlusselemente 102 bis an die Löselaschenhauptfaltlinie 56 heran, wie dies schematisch in Figur 11 sowie in den Figuren 14 und 15 dargestellt ist.

Ein freies, in Richtung auf die Löselaschenhauptfaltlinie 56 hin weisendes, klebstofffreies Zugende 110 der Klebestreifen 106 kann von einem Anwender erfasst und so der Klebestreifen 106 auseinandergezogen werden. Ein dem Zugende 110 entgegengesetztes Befestigungsende 112 des Klebestreifens 106 ist dauerhaft mit der zweiten Flachmaterialseitenfläche 32 verbunden. Das wie beschrieben ausgezogene Verschlusselement 102 kann dann über die Löselaschenhauptfaltlinie 56 hinausgezogen und um die Verpackungsvorrichtung 10 herumgeführt und an dieser festgelegt werden, wie dies in Figur 12 schematisch dargestellt ist.

Bei einem weiteren Ausführungsbeispiel sind alternativ oder zusätzlich zwei Verschlusselemente vorgesehen. Diese sind in den Figuren 11 und 12 schematisch eingezeichnet und mit dem Bezugszeichen 104 bezeichnet. Die Verschlusselemente 104 sind entsprechend den Klebestreifen 106 ausgebildet und definieren jeweils eine Verschlusselementlängsrichtung 114, die sich parallel zur Hauptfaltlinie 24 erstreckt. In der Einführstellung erstrecken sich die Verschlusselemente 104 bis an eine der beiden Seitenfaltlinien 26 beziehungsweise 28 heran und stehen in der Verschlussstellung jeweils über eine der beiden Seitenfaltlinien 26 und 28 vor, um das umgeschlagene freie Ende 98 in der Verschlussstellung wie beschrieben und in Figur 12 dargestellt seitlich zu fixieren.

Die in den Figuren dargestellten und beschriebenen Ausführungsbeispiele sind aus einem Flachmaterialbogen 18 ausgebildet, welcher insbesondere ein Tuch, ein Vlies, beispielsweise ein Kunststoffvlies, oder ein Verpackungspapier, zum Beispiel ein Krepppapier, sein kann.

Die Verpackungsvorrichtung 10 ist in Form eines Einwegprodukts ausgebildet. Ferner ist sie sterilisierbar, insbesondere heißdampfsterilisierbar.

Wie beschrieben ist die Verpackungsvorrichtung 10 aus einem einstückigen, nämlich einem monolithischen, Flachmaterialbogen 18 ausgebildet.

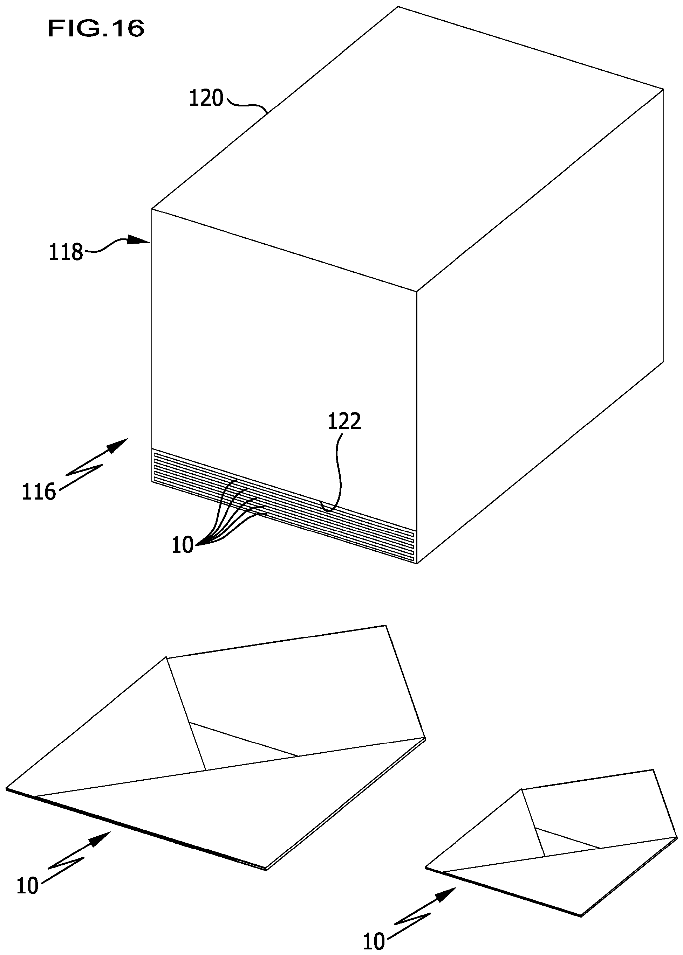

In Figur 16 ist schematisch ein Ausführungsbeispiel eines medizinischen Verpackungssystems 116 dargestellt. Es umfasst mehrere medizinische Verpackungsvorrichtungen 10.

Das Verpackungssystem 116 umfasst eine Mehrzahl medizinischer Verpackungsvorrichtungen 10, die sich in Form und Größe sowie wahlweise auch durch den Werkstoff, aus dem der Flachmaterialbogen 18 ausgebildet ist, voneinander unterscheiden.

Das in Figur 16 dargestellte Ausführungsbeispiel umfasst eine Umverpackung 118 für eine Mehrzahl von Verpackungsvorrichtungen 10. Die Umverpackung 118 ist in Form einer Spendervorrichtung 120 mit einer Entnahmeöffnung 122 ausgebildet. Durch die Entnahmeöffnung 122 können Verpackungsvorrichtungen 10 aus der Spendervorrichtung 120 entnommen werden.

Das Verpackungssystem 116 umfasst optional weitere Spendervorrichtungen 120, in welchen jeweils identische Verpackungsvorrichtungen 10 aufgenommen sind. Sind beispielsweise drei unterschiedliche Größen von Siebkörben 12 zu verpacken, sind vorzugsweise drei Spendervorrichtungen 120 bereitgestellt, die jeweils eine Mehrzahl identischer Verpackungsvorrichtungen 10 enthalten, wobei die Spendervorrichtungen 120 an die Größe und Form der Verpackungsvorrichtungen 10 für die drei unterschiedlichen Siebkörbe 12 angepasst sind. Mithin können demnach beispielsweise drei unterschiedlich große Spendervorrichtungen 120 bereitgestellt werden.

Die beschriebenen Ausführungsbeispiele medizinischer Verpackungsvorrichtungen 10 sowie die beschriebenen Ausführungsbeispiele von Verpackungssystemen 116 ermöglichen es insbesondere, Siebkörbe 12 schnell und effizient zu verpacken. Insbesondere ist es nicht erforderlich, einem Anwender eine bestimmte Falttechnik zum Einschlagen des Siebkorbs 12 in einen Flachmaterialbogen 18 beizubringen. Vielmehr kann ein Anwender den Siebkorb 12 einfach durch die Einführöffnung 16 in den Aufnahmeraum 14 der Verpackungsvorrichtung 10 einschieben und diese dann durch Umschlagen des freien Endes 98 über die Einführöffnung 16 verschließen. Auf diese Weise kann eine signifikante Zeitersparnis beim Verpacken der Siebkörbe 12 mit Verpackungsvorrichtungen 10 in Form von Weichverpackungen erreicht werden, woraus sich auch eine Kostenersparnis ergibt.

Durch die vorkonfektioniert bereitgestellten Verpackungsvorrichtungen 10 kann ferner eine Reproduzierbarkeit bei der Verpackung von Siebkörben 12 signifikant erhöht werden. Zudem verringert sich ein Schulungsaufwand für Personal, das die Siebkörbe 12 verpackt, deutlich.

### Bezugszeichenliste

- 10: Verpackungsvorrichtung
- 12: Siebkorb
- 14: Aufnahmeraum
- 16: Einführöffnung
- 18: Flachmaterialbogen
- 20: erster Aufnahmeraumflachmaterialbogenflächenbereich
- 22: zweiter Aufnahmeraumflachmaterialbogenflächenbereich
- 24: Hauptfaltlinie
- 26: erste Seitenfaltlinie
- 28: zweite Seitenfaltlinie
- 30: erste Flachmaterialseitenfläche
- 32: zweite Flachmaterialseitenfläche
- 34: erste Hauptecke
- 36: zweite Hauptecke
- 38: Abstand
- 40: Abstand
- 42: erste Nebenecke
- 44: zweite Nebenecke
- 46: erste Löselasche
- 48: Nebenfaltlinie
- 50: Abstand
- 52: Abstand
- 54: zweite Löselasche
- 56: Löselaschenhauptfaltlinie
- 58: Löselaschennebenfaltlinie
- 60: Abstand
- 62: Abstand
- 64: Abstand
- 66: Abstand
- 68: Entfaltungssicherungseinrichtung
- 70: erstes Sicherungselement
- 72: erster Sicherungsflächenbereich
- 74: zweiter Sicherungsflächenbereich
- 76: Seitenkante
- 78: Seitenkante
- 80: Seitenkante
- 82: Seitenkante
- 84: Spitze
- 86: dreieckige Fläche
- 88: Klebestreifen
- 90: Klebeelement
- 92: erste Klebefläche
- 94: zweite Klebefläche
- 96: Schutzfolie
- 98: freies Ende
- 100: Verschlussfaltlinie
- 102: erstes Verschlusselement
- 104: zweites Verschlusselement
- 106: Klebestreifen
- 108: erste Verschlusselementlängsrichtung
- 110: Zugende
- 112: Befestigungsende
- 114: zweite Verschlusselementlängsrichtung
- 116: Verpackungssystem
- 118: Umverpackung
- 120: Spendervorrichtung
- 122: Entnahmeöffnung
- 170: zweites Sicherungselement
- 172: erster Sicherungsflächenbereich
- 174: zweiter Sicherungsflächenbereich

## Patentansprüche

1. Medizinische Verpackungsvorrichtung (10) zum sterilen Verpacken eines Siebkorbs (12), wobei die Verpackungsvorrichtung (10) einen Aufnahmeraum (14) zum Aufnehmen eines Siebkorbs (12) in einer Verpackungsstellung und eine Einführöffnung (16) zum Einführen eines Siebkorbs (12) in den Aufnahmeraum (14) hinein definiert, wobei die Einführöffnung (16) in einer Einführstellung offen ist, wobei die Verpackungsvorrichtung (10) aus einem in einer Ausgangsstellung ungefalteten Flachmaterialbogen (18) durch mehrfaches Falten gebildet ist, wobei der Aufnahmeraum (14) in der Einführstellung zwei aneinander anliegende Aufnahmeraumflachmaterialbogenflächenbereiche (20, 22) aufweist, welche durch mindestens drei Faltlinien (24, 26, 28) des Flachmaterialbogens (18) begrenzt sind derart, dass der Aufnahmeraum (14) bis auf die Einführöffnung (16) allseitig geschlossen ist, **dadurch gekennzeichnet, dass** die Verpackungsvorrichtung (10) eine Entfaltungssicherungseinrichtung (68) umfasst zum Sichern der Verpackungsvorrichtung (10) in der Einführstellung gegen ein vollständiges Entfalten zurück in die Ausgangsstellung.

2. Medizinische Verpackungsvorrichtung nach Anspruch 1, **dadurch gekennzeichnet, dass**
a) der Flachmaterialbogen (18) eine erste Flachmaterialseitenfläche (30) und eine zweite Flachmaterialseitenfläche (32) definiert, dass die erste Flachmaterialseitenfläche (30) und die zweite Flachmaterialseitenfläche (32) in der Ausgangsstellung in voneinander entgegengesetzte Richtungen weisen und dass die erste Flachmaterialseitenfläche (30) die zwei Aufnahmeraumflachmaterialbogenflächenbereiche (20, 22) umfasst
und/oder
b) die Verpackungsvorrichtung (10) in der Einführstellung mindestens drei Faltlinien (24, 26, 28), insbesondere drei, vier, fünf, sechs oder sieben Faltlinien, umfasst
und/oder
c) der Flachmaterialbogen (18) in der Ausgangsstellung viereckig, insbesondere rechteckig, weiter insbesondere quadratisch oder im Wesentlichen quadratisch, ausgebildet ist
und/oder
d) eine der mindestens drei Faltlinien in Form einer Hauptfaltlinie (24) ausgebildet ist und dass die Hauptfaltlinie (24) zwei in der Ausgangsstellung einander gegenüberliegende Hauptecken (34, 36) des Flachmaterialbogens (18) miteinander verbindet.

3. Medizinische Verpackungsvorrichtung nach einem der voranstehenden Ansprüche, **dadurch gekennzeichnet, dass** der Flachmaterialbogen (18) in der Ausgangsstellung zwei einander gegenüberliegende Nebenecken (42, 44) umfasst, dass die zwei Nebenecken (42, 44) in einer ersten Faltstellung, in welcher der Flachmaterialbogen (18) von der Ausgangsstellung um die Hauptfaltlinie (24) gefaltet ist, aufeinander liegen, dass die Verpackungsvorrichtung (10) eine erste Löselasche (46) umfasst, dass die erste Löselasche (46) durch Zurückfalten einer ersten der zwei Nebenecken (42, 44) aus der ersten Faltstellung in eine zweite Faltstellung um eine Nebenfaltlinie (48) ausgebildet ist und dass die Nebenfaltlinie (48) parallel oder im Wesentlichen parallel zur Hauptfaltlinie (24) verläuft und sich zwischen der Hauptfaltlinie (24) und den Nebenecken (42, 44) in der ersten Faltstellung erstreckt.

4. Medizinische Verpackungsvorrichtung nach Anspruch 3, **dadurch gekennzeichnet, dass** eine erste der zwei Hauptecken (34, 36) von der zweiten Faltstellung um eine erste der zwei Seitenfaltlinien (26, 28) in Richtung auf eine zweite der zwei Hauptecken (34, 36) hin in eine dritte Faltstellung umgefaltet ist und dass die zweite der zwei Hauptecken (34, 36) von der dritten Faltstellung in eine vierte Faltstellung um eine zweite der zwei Seitenfaltlinien (26, 28) in Richtung auf die erste der zwei Seitenfaltlinien (26, 28) hin umgefaltet ist.

5. Medizinische Verpackungsvorrichtung nach einem der voranstehenden Ansprüche, **dadurch gekennzeichnet, dass** die Entfaltungssicherungseinrichtung (68) mindestens ein Sicherungselement (70, 170) umfasst zum Sichern der Verpackungsvorrichtung (10) in der Einführstellung, wobei insbesondere
a) die Verpackungsvorrichtung (10) mindestens einen ersten Sicherungsflächenbereich (72, 172) und mindestens einen zweiten Sicherungsflächenbereich (74, 174) definiert, wobei der erste Sicherungsflächenbereich (72, 172) und der zweite Sicherungsflächenbereich (74, 174) von den zwei in der Einführstellung aneinander anliegenden Aufnahmeraumflachmaterialbogenflächenbereichen (20, 22) verschieden sind, wobei der erste Sicherungsflächenbereich (72, 172) und der zweite Sicherungsflächenbereich (74, 174) in der Einführstellung aneinander anliegen und wobei das mindestens eine Sicherungselement (70, 170) den mindestens einen ersten Sicherungsflächenbereich (72, 172) und den mindestens einen zweiten Sicherungsflächenbereich (74, 174) kraft- und/oder stoffschlüssig miteinander verbindet, insbesondere durch Kleben und/oder Schweißen,
und/oder
b) der mindestens eine erste Sicherungsflächenbereich (72, 172) und der zugeordnete mindestens eine zweite Sicherungsflächenbereich (74, 174) von derselben Flachmaterialseitenfläche (30, 32) oder von unterschiedlichen Flachmaterialseitenflächen (30, 32) umfasst sind
und/oder
c) das mindestens eine Sicherungselement (70, 170) durch einen Schweißpunkt, durch einen Klebstoff oder durch ein Klebeelement (90) ausgebildet ist, wobei insbesondere das Klebeelement (90) zwei voneinander weg weisende Klebeflächen (92, 94) aufweist und wobei in der Einführstellung die eine der zwei Klebeflächen (92, 94) am mindestens einen ersten Sicherungsflächenbereich (72, 172) anliegt und wobei die andere der zwei Klebeflächen (92, 94) am mindestens einen zweiten Sicherungsflächenbereich (72, 172) anliegt,
und/oder
d) das mindestens eine Sicherungselement (70, 170) in einem Bereich der Verpackungsvorrichtung (10) positioniert ist, in welchem in der Einführstellung mindestens zwei durch Umfalten des Flachmaterialbogens (18) ausgebildete Lagen aufeinanderliegen.

6. Medizinische Verpackungsvorrichtung nach einem der voranstehenden Ansprüche, **dadurch gekennzeichnet, dass** die Verpackungsvorrichtung (10) mindestens ein Verschlusselement (102, 104) umfasst zum Verschließen der Verpackungsvorrichtung (10) in einer Verpackungsstellung, in welcher die Einführöffnung (16) verschlossen ist durch Umfalten der zweiten Nebenecke (44) um eine Verschlussfaltlinie (100) in Richtung auf die erste Nebenecke (42) hin.

7. Medizinische Verpackungsvorrichtung nach einem der voranstehenden Ansprüche, **dadurch gekennzeichnet, dass**
a) der Flachmaterialbogen (18) aus einem Tuch, einem Vlies, insbesondere einem Kunststoffvlies, oder einem Verpackungspapier, insbesondere einem Krepppapier, ausgebildet ist und/oder
b) die Verpackungsvorrichtung (10) in Form eines Einwegprodukts ausgebildet ist
und/oder
c) die Verpackungsvorrichtung (10) sterilisierbar, insbesondere heißdampfsterilisierbar, ausgebildet ist
und/oder
d) die Verpackungsvorrichtung (10) aus einem einstückigen, insbesondere monolithischen, Flachmaterialbogen (18) ausgebildet ist.

8. Medizinisches, insbesondere steriles, Verpackungssystem (116) für Siebkörbe (12) umfassend eine Mehrzahl von medizinischen Verpackungsvorrichtungen (10) nach einem der voranstehenden Ansprüche,
wobei insbesondere
a) sich mindestens zwei der Mehrzahl von medizinischen Verpackungsvorrichtungen (10) in Form und/oder Größe und/oder durch den Werkstoff, aus dem der Flachmaterialbogen (18) ausgebildet ist, voneinander unterscheiden
und/oder
b) das Verpackungssystem (116) eine Umverpackung (118) für die Mehrzahl von Verpackungsvorrichtungen (10) umfasst, insbesondere in Form einer Spendervorrichtung (120) mit einer Entnahmeöffnung (122), durch die hindurch Verpackungsvorrichtungen (10) einzeln aus der Spendervorrichtung (120) entnehmbar sind,
und/oder
c) das Verpackungssystem (116) mindestens einen Siebkorb (12) umfasst.

9. Verfahren zum Herstellen einer medizinischen Verpackungsvorrichtung (10) zum sterilen Verpacken eines Siebkorbs (12), wobei die Verpackungsvorrichtung (10) ausgebildet wird mit einem Aufnahmeraum (14) zum Aufnehmen eines Siebkorbs (12) in einer Verpackungsstellung und einer Einführöffnung (16) zum Einführen eines Siebkorbs (12) in den Aufnahmeraum (14) hinein, wobei die Einführöffnung (16) in einer Einführstellung offen ist, wobei die Verpackungsvorrichtung (10) aus einem in einer Ausgangsstellung ungefalteten Flachmaterialbogen (18) durch mehrfaches Falten gebildet wird, wobei der Aufnahmeraum (14) in der Einführstellung zwei aneinander anliegende Aufnahmeraumflachmaterialbogenflächenbereiche (20, 22) aufweist, welche durch mindestens drei Faltlinien (24, 26, 28) des Flachmaterialbogens (18) begrenzt sind derart, dass der Aufnahmeraum (14) bis auf die Einführöffnung (16) allseitig geschlossen ist, **dadurch gekennzeichnet, dass** die Verpackungsvorrichtung (10) in der Einführstellung gegen ein vollständiges Entfalten zurück in die Ausgangsstellung gesichert wird.

10. Verfahren nach Anspruch 9, **dadurch gekennzeichnet, dass**
a) der Flachmaterialbogen (18) eine erste Flachmaterialseitenfläche (30) und eine zweite Flachmaterialseitenfläche (32) definiert, dass die erste Flachmaterialseitenfläche (30) und die zweite Flachmaterialseitenfläche (32) in der Ausgangsstellung in voneinander entgegengesetzte Richtungen weisen und dass die zwei Aufnahmeraumflachmaterialbogenflächenbereiche (20, 22) durch Falten des Flachmaterialbogens (18) aus der ersten Flachmaterialseitenfläche (30) gebildet werden
und/oder
b) die Verpackungsvorrichtung (10) durch Falten des Flachmaterialbogens (18) entlang von mindestens drei Faltlinien (24, 26, 28) gebildet wird, insbesondere entlang von drei, vier, fünf, sechs oder sieben Faltlinien,
und/oder
c) ein Flachmaterialbogen (18) bereitgestellt wird, welcher in der Ausgangsstellung viereckig, insbesondere rechteckig, weiter insbesondere quadratisch oder im Wesentlichen quadratisch ausgebildet ist,
und/oder
d) eine der mindestens drei Faltlinien in Form einer Hauptfaltlinie (24) ausgebildet wird, welche zwei in der Ausgangsstellung einander gegenüberliegende Hauptecken (34, 36) des Flachmaterialbogens (18) miteinander verbindet.

11. Verfahren nach Anspruch 9 oder 10, **dadurch gekennzeichnet, dass** der Flachmaterialbogen (18) in der Ausgangsstellung zwei einander gegenüberliegende Nebenecken (42, 44) umfasst, dass die zwei Nebenecken (42, 44) durch Falten des Flachmaterialbogens (18) von der Ausgangsstellung um die Hauptfaltlinie (24) in eine erste Faltstellung gebracht werden, in welcher sie aufeinander liegen, dass eine erste Löselasche (46) der Verpackungsvorrichtung (10) ausgebildet wird durch Zurückfalten einer ersten der zwei Nebenecken (42, 44) um eine Nebenfaltlinie (48) aus der ersten Faltstellung in eine zweite Faltstellung und dass die Nebenfaltlinie (48) parallel oder im Wesentlichen parallel zur Hauptfaltlinie (24) verläuft und sich zwischen der Hauptfaltlinie (24) und den Nebenecken (42, 44) in der ersten Faltstellung erstreckt,
wobei insbesondere
a) die erste Löselasche (46) in der Einführstellung über die Hauptfaltlinie (24) vorstehend ausgebildet wird
und/oder
b) die Nebenfaltlinie (48) ausgebildet wird mit einem Abstand (50) von der Hauptfaltlinie (24), welcher kleiner ist als ein Abstand (52) von den Nebenecken (42, 44) in der ersten Faltstellung,
und/oder
c) die Nebenfaltlinie (48) die Einführöffnung (16) begrenzend ausgebildet wird.

12. Verfahren nach Anspruch 11, **dadurch gekennzeichnet, dass** eine erste der zwei Hauptecken (34, 36) von der zweiten Faltstellung um eine erste der zwei Seitenfaltlinien (26, 28) in Richtung auf eine zweite der zwei Hauptecken (34, 36) hin in eine dritte Faltstellung umgefaltet wird und dass die zweite der zwei Hauptecken (34, 36) von der dritten Faltstellung in eine vierte Faltstellung um eine zweite der zwei Seitenfaltlinien (26, 28) in Richtung auf die erste der zwei Seitenfaltlinien (26, 28) hin umgefaltet wird,
wobei insbesondere die Verpackungsvorrichtung (10) derart ausgebildet wird, dass die vierte Faltstellung die Einführstellung definiert.

13. Verfahren nach Anspruch 12, **dadurch gekennzeichnet, dass** eine zweite Löselasche (54) der Verpackungsvorrichtung (10) ausgebildet wird durch Umfalten einer zweiten der zwei Nebenecken (42, 44), welche in der Einführstellung von der Einführöffnung (16) weg weist, von der vierten Faltstellung in eine fünfte Faltstellung um eine Löselaschenhauptfaltlinie (56) in Richtung auf die Hauptfaltlinie (24) hin und durch Zurückfalten der zweiten Nebenecke (44) von der fünften Faltstellung in eine sechste Faltstellung um eine Löselaschennebenfaltlinie (58) in einer Richtung von der Hauptfaltlinie (24) weg.

14. Verfahren nach einem der Ansprüche 9 bis 13, **dadurch gekennzeichnet, dass** die Verpackungsvorrichtung (10) mit mindestens einem Sicherungselement (70, 170) in der Einführstellung gesichert wird,
wobei insbesondere
a) die Verpackungsvorrichtung (10) mindestens einen ersten Sicherungsflächenbereich (72, 172) und mindestens einen zweiten Sicherungsflächenbereich (74, 174) definiert, wobei der erste Sicherungsflächenbereich (72, 172) und der zweite Sicherungsflächenbereich (74, 174) von den zwei in der Einführstellung aneinander anliegenden Aufnahmeraumflachmaterialbogenflächenbereichen (20, 22) verschieden sind, wobei der erste Sicherungsflächenbereich (72, 172) und der zweite Sicherungsflächenbereich (74, 174) in der Einführstellung aneinander anliegen und wobei mit dem mindestens einen Sicherungselement (70, 170) der mindestens eine erste Sicherungsflächenbereich (72, 172) und der mindestens eine zweite Sicherungsflächenbereich (74, 174) kraft- und/oder stoffschlüssig miteinander verbunden werden, insbesondere durch Kleben und/oder Schweißen,
wobei weiter insbesondere die Verpackungsvorrichtung (10) derart ausgebildet wird, dass der mindestens eine erste Sicherungsflächenbereich (72, 172) und der zugeordnete mindestens eine zweite Sicherungsflächenbereich (74, 174) von derselben Flachmaterialseitenfläche (30, 32) oder von unterschiedlichen Flachmaterialseitenflächen (30, 32) umfasst sind,
und/oder
b) das mindestens eine Sicherungselement (70, 170) durch einen Schweißpunkt, durch einen Klebstoff oder durch ein Klebeelement (90) ausgebildet wird, wobei insbesondere das Klebeelement (90) zwei voneinander weg weisende Klebeflächen (92, 94) aufweist und derart angebracht wird, insbesondere in der Ausgangsstellung, dass in der Einführstellung die eine der zwei Klebeflächen (92, 94) am mindestens einen ersten Sicherungsflächenbereich (72, 172) anliegt und dass die andere der zwei Klebeflächen (92, 94) am mindestens einen zweiten Sicherungsflächenbereich (74, 174) anliegt,
und/oder
c) das mindestens eine Sicherungselement (70, 170) in einem Bereich der Verpackungsvorrichtung (10) positioniert wird, in welchem in der Einführstellung mindestens zwei durch Umfalten des Flachmaterialbogens (18) ausgebildete Lagen aufeinanderliegen.

15. Verfahren nach einem der Ansprüche 9 bis 14, **dadurch gekennzeichnet, dass**
a) der Flachmaterialbogen (18) aus einem Tuch, einem Vlies, insbesondere einem Kunststoffvlies, oder einem Verpackungspapier, insbesondere einem Krepppapier, ausgebildet wird
und/oder
b) die Verpackungsvorrichtung (18) in Form eines Einwegprodukts ausgebildet wird
und/oder
c) die Verpackungsvorrichtung (18) sterilisierbar, insbesondere heißdampfsterilisierbar, ausgebildet wird
und/oder
d) die Verpackungsvorrichtung (10) aus einem einstückigen, insbesondere monolithischen, Flachmaterialbogen (18) ausgebildet wird.

## Claims

1. Medical packaging device (10) for sterile packaging of a sieve basket (12), wherein the packaging device (10) defines a receiving space (14) for accommodating a sieve basket (12) in a packaging position and an insertion opening (16) for inserting a sieve basket (12) into the receiving space (14), wherein the insertion opening (16) is open in an insertion position, wherein the packaging device (10) is made from a flat material sheet (18) that is unfolded in a starting position by folding multiple times, wherein the receiving space (14) in the insertion position has two abutting receiving space flat material sheet surface regions (20, 22), which are delimited by at least three fold lines (24, 26, 28) of the flat material sheet (18) in such a way that the receiving space (14) is closed on all sides except for the insertion opening (16), **characterized in that** the packaging device (10) comprises an unfolding securing device (68) for securing the packaging device (10) in the insertion position against being completely unfolded back into the starting position.

2. Medical packaging device in accordance with Claim 1, **characterized in that**
a) the flat material sheet (18) defines a first flat material side face (30) and a second flat material side face (32), **in that** the first flat material side face (30) and the second flat material side face (32) face in opposite directions in the starting position, and **in that** the first flat material side face (30) comprises the two receiving space flat material sheet surface regions (20, 22),
and/or
b) the packaging device (10) in the insertion position comprises at least three fold lines (24, 26, 28), in particular three, four, five, six, or seven fold lines,
and/or
c) the flat material sheet (18) in the starting position is of quadrangular, in particular rectangular, further particularly square or substantially square configuration,
and/or
d) one of the at least three fold lines is configured in the form of a main fold line (24) and **in that** the main fold line (24) connects two main corners (34, 36) of the flat material sheet (18) that are located opposite one another in the starting position.

3. Medical packaging device in accordance with any one of the preceding Claims, **characterized in that** the flat material sheet (18) in the starting position comprises two opposing secondary corners (42, 44), **in that** the two secondary corners (42, 44) lie on one another in a first folded position in which the flat material sheet (18) is folded from the starting position about the main fold line (24), **in that** the packaging device (10) comprises a first release tab (46), **in that** the first release tab (46) is formed by folding back a first one of the two secondary corners (42, 44) from the first folded position into a second folded position about a secondary fold line (48), and **in that** the secondary fold line (48) runs in parallel or substantially in parallel to the main fold line (24) and extends between the main fold line (24) and the secondary corners (42, 44) in the first folded position.

4. Medical packaging device in accordance with Claim 3, **characterized in that** a first one of the two main corners (34, 36) is folded from the second folded position about a first one of the two side fold lines (26, 28) in the direction toward a second one of the two main corners (34, 36) into a third folded position and **in that** the second one of the two main corners (34, 36) is folded from the third folded position into a fourth folded position about a second one of the two side fold lines (26, 28) in the direction toward the first one of the two side fold lines (26, 28).

5. Medical packaging device in accordance with any one of the preceding Claims, **characterized in that** the unfolding securing device (68) comprises at least one securing element (70, 170) for securing the packaging device (10) in the insertion position,
wherein, in particular,
a) the packaging device (10) defines at least one first securing surface region (72, 172) and at least one second securing surface region (74, 174), wherein the first securing surface region (72, 172) and the second securing surface region (74, 174) are different from the two receiving space flat material sheet surface regions (20, 22) that abut against one another in the insertion position, wherein the first securing surface region (72, 172) and the second securing surface region (74, 174) abut against one another in the insertion position, and wherein the at least one securing element (70, 170) connects the at least one first securing surface region (72, 172) and the at least one second securing surface region (74, 174) to one another in a force-locking and/or materially bonded manner, in particular by adhesion and/or welding,
and/or
b) the at least one first securing surface region (72, 172) and the associated at least one second securing surface region (74, 174) are comprised by the same flat material side face (30, 32) or by different flat material side faces (30, 32),
and/or
c) the at least one securing element (70, 170) is formed by a welding point, by an adhesive, or by an adhesive element (90), wherein, in particular, the adhesive element (90) has two adhesive surfaces (92, 94) that face away from one another, and wherein in the insertion position the one of the two adhesive surfaces (92, 94) abuts against the at least one first securing surface region (72, 172) and wherein the other one of the two adhesive surfaces (92, 94) abuts against the at least one second securing surface region (72, 172),
and/or
d) the at least one securing element (70, 170) is positioned in a region of the packaging device (10) in which at least two layers formed by folding the flat material sheet (18) lie on one another in the insertion position.

6. Medical packaging device in accordance with any one of the preceding Claims, **characterized in that** the packaging device (10) comprises at least one closure element (102, 104) for closing the packaging device (10) in a packaging position in which the insertion opening (16) is closed by folding the second secondary corner (44) about a closure fold line (100) in the direction toward the first secondary corner (42).

7. Medical packaging device in accordance with any one of the preceding Claims, **characterized in that**
a) the flat material sheet (18) is made of a cloth, a non-woven fabric, in particular a non-woven plastic fabric, or a packaging paper, in particular a crepe paper,
and/or
b) the packaging device (10) is configured in the form of a disposable product,
and/or
c) the packaging device (10) is configured to be sterilizable, in particular sterilizable with hot steam,
and/or
d) the packaging device (10) is made from a one-piece, in particular monolithic, flat material sheet (18).

8. Medical, in particular sterile, packaging system (116) for sieve baskets (12) comprising a plurality of medical packaging devices (10) in accordance with any one of the preceding Claims,
wherein, in particular,
a) at least two of the plurality of medical packaging devices (10) differ from one another in shape and/or size and/or in the material of which the flat material sheet (18) is made,
and/or
b) the packaging system (116) comprises an outer packaging (118) for the plurality of packaging devices (10), in particular in the form of a dispenser device (120) with a removal opening (122) through which packaging devices (10) are individually removable from the dispenser device (120),
and/or
c) the packaging system (116) comprises at least one sieve basket (12).

9. Method for producing a medical packaging device (10) for sterile packaging of a sieve basket (12), wherein the packaging device (10) is configured with a receiving space (14) for accommodating a sieve basket (12) in a packaging position and an insertion opening (16) for inserting a sieve basket (12) into the receiving space (14), wherein the insertion opening (16) is open in an insertion position, wherein the packaging device (10) is made from a flat material sheet (18) that is unfolded in a starting position by folding multiple times, wherein the receiving space (14) in the insertion position has two abutting receiving space flat material sheet surface regions (20, 22), which are delimited by at least three fold lines (24, 26, 28) of the flat material sheet (18) in such a way that the receiving space (14) is closed on all sides except for the insertion opening (16), **characterized in that** the packaging device (10) is secured in the insertion position against being completely unfolded back into the starting position.

10. Method in accordance with Claim 9, **characterized in that**
a) the flat material sheet (18) defines a first flat material side face (30) and a second flat material side face (32), **in that** the first flat material side face (30) and the second flat material side face (32) face in opposite directions in the starting position, and **in that** the two receiving space flat material sheet surface regions (20, 22) are formed from the first flat material side face (30) by folding the flat material sheet (18),
and/or
b) the packaging device (10) is formed by folding the flat material sheet (18) along at least three fold lines (24, 26, 28), in particular along three, four, five, six, or seven fold lines,
and/or
c) a flat material sheet (18) is provided, which in the starting position is of quadrangular, in particular rectangular, further particularly square or substantially square configuration, and/or
d) one of the at least three fold lines is configured in the form of a main fold line (24), which connects two main corners (34, 36) of the flat material sheet (18) that are located opposite one another in the starting position.

11. Method in accordance with Claim 9 or 10, **characterized in that** the flat material sheet (18) in the starting position comprises two opposing secondary corners (42, 44), **in that** the two secondary corners (42, 44) are brought into a first folded position by folding the flat material sheet (18) from the starting position about the main fold line (24), in which first folded position they lie on one another, **in that** a first release tab (46) of the packaging device (10) is formed by folding back a first one of the two secondary corners (42, 44) about a secondary fold line (48) from the first folded position into a second folded position, and **in that** the secondary fold line (48) runs in parallel or substantially in parallel to the main fold line (24) and extends between the main fold line (24) and the secondary corners (42, 44) in the first folded position,
wherein, in particular,
a) the first release tab (46) in the insertion position is configured projecting over the main fold line (24),
and/or
b) the secondary fold line (48) is configured at a distance (50) from the main fold line (24) that is smaller than a distance (52) from the secondary corners (42, 44) in the first folded position,
and/or
c) the secondary fold line (48) is configured delimiting the insertion opening (16).

12. Method in accordance with Claim 11, **characterized in that** a first one of the two main corners (34, 36) is folded from the second folded position about a first one of the two side fold lines (26, 28) in the direction toward a second one of the two main corners (34, 36) into a third folded position and **in that** the second one of the two main corners (34, 36) is folded from the third folded position into a fourth folded position about a second one of the two side fold lines (26, 28) in the direction toward the first one of the two side fold lines (26, 28),
wherein, in particular, the packaging device (10) is configured in such a way that the fourth folded position defines the insertion position.

13. Method in accordance with Claim 12, **characterized in that** a second release tab (54) of the packaging device (10) is formed by folding a second one of the two secondary corners (42, 44), which in the insertion position points away from the insertion opening (16), from the fourth folded position into a fifth folded position about a release tab main fold line (56) in the direction toward the main fold line (24) and by folding back the second secondary corner (44) from the fifth folded position into a sixth folded position about a release tab secondary fold line (58) in a direction away from the main fold line (24).

14. Method in accordance with any one of Claims 9 to 13, **characterized in that** the packaging device (10) is secured in the insertion position with at least one securing element (70, 170),
wherein, in particular,
a) the packaging device (10) defines at least one first securing surface region (72, 172) and at least one second securing surface region (74, 174), wherein the first securing surface region (72, 172) and the second securing surface region (74, 174) are different from the two receiving space flat material sheet surface regions (20, 22) that abut against one another in the insertion position, wherein the first securing surface region (72, 172) and the second securing surface region (74, 174) abut against one another in the insertion position, and wherein the at least one first securing surface region (72, 172) and the at least one second securing surface region (74, 174) are connected to one another in a force-locking and/or materially bonded manner, in particular by adhesion and/or welding, with the at least one securing element (70, 170), wherein, further in particular, the packaging device (10) is configured in such a way that the at least one first securing surface region (72, 172) and the associated at least one second securing surface region (74, 174) are comprised by the same flat material side face (30, 32) or by different flat material side faces (30, 32),
and/or
b) the at least one securing element (70, 170) is formed by a welding point, by an adhesive, or by an adhesive element (90), wherein, in particular, the adhesive element (90) has two adhesive faces (92, 94) that face away from one another and is attached in such a way, in particular in the starting position, that in the insertion position the one of the two adhesive surfaces (92, 94) abuts against the at least one first securing surface region (72, 172) and that the other one of the two adhesive surfaces (92, 94) abuts against the at least one second securing surface region (74, 174),
and/or
c) the at least one securing element (70, 170) is positioned in a region of the packaging device (10) in which at least two layers formed by folding the flat material sheet (18) lie on one another in the insertion position.

15. Method in accordance with any one of Claims 9 to 14, **characterized in that**
a) the flat material sheet (18) is made of a cloth, a non-woven fabric, in particular a non-woven plastic fabric, or a packaging paper, in particular a crepe paper,
and/or
b) the packaging device (18) is configured in the form of a disposable product,
and/or
c) the packaging device (18) is configured to be sterilizable, in particular sterilizable with hot steam,
and/or
d) the packaging device (10) is made from a one-piece, in particular monolithic, flat material sheet (18).

## Revendications

1. Dispositif d'emballage médical (10) pour l'emballage stérile d'un panier perforé (12), le dispositif d'emballage (10) définissant un espace de réception (14) pour héberger un panier perforé (12) dans une position d'emballage et une ouverture d'insertion (16) pour l'insertion d'un panier perforé (12) dans l'espace de réception (14), l'ouverture d'insertion (16) étant ouverte dans une position d'insertion, le dispositif d'emballage (10) étant formé par pliage multiple d'une feuille de matériau plat (18) non pliée dans une position initiale, l'espace de réception (14) présentant dans la position d'insertion deux zones de surface de feuille de matériau plat de l'espace de réception (20, 22) adjacentes l'une à l'autre, qui sont délimitées par au moins trois lignes de pliage (24, 26, 28) de la feuille de matériau plat (18) de manière à ce que l'espace de réception (14) soit fermé de tous les côtés à l'exception de l'ouverture d'insertion (16), **caractérisé en ce que** le dispositif d'emballage (10) comprend un dispositif de sécurité anti-déploiement (68) pour empêcher le dispositif d'emballage (10) en position d'insertion de se déployer complètement pour revenir dans sa position initiale.

2. Dispositif d'emballage médical selon la revendication 1, **caractérisé en ce que**
a) la feuille de matériau plat (18) définit une première surface latérale de matériau plat (30) et une deuxième surface latérale de matériau plat (32), que la première surface latérale de matériau plat (30) et la deuxième surface latérale de matériau plat (32) sont orientées dans des directions opposées l'une à l'autre dans la position initiale et que la première surface latérale de matériau plat (30) comprend les deux zones de surface de feuille de matériau plat de l'espace de réception (20, 22)
et/ou
b) le dispositif d'emballage (10) comprend, en position d'insertion, au moins trois lignes de pliage (24, 26, 28), en particulier trois, quatre, cinq, six ou sept lignes de pliage
et/ou
c) la feuille de matériau plat (18) est de forme carrée, en particulier rectangulaire, en outre en particulier voire carrée ou quasiment carrée, dans sa position initiale
et/ou
d) l'une des trois lignes de pliage au moins est conçue sous la forme d'une ligne de pliage principale (24) et que la ligne de pliage principale (24) relie entre eux deux coins principaux (34, 36) de la feuille de matériau plat (18) qui sont opposés l'un à l'autre dans la position initiale.

3. Dispositif d'emballage médical selon l'une des revendications précédentes, **caractérisé en ce que** la feuille de matériau plat (18) comprend, dans la position initiale, deux coins secondaires opposés (42, 44), que les deux coins secondaires (42, 44) se superposent dans une première position de pliage, dans laquelle la feuille de matériau plat (18) est pliée à partir de la position initiale autour de la ligne de pliage principale (24), que le dispositif d'emballage (10) comprend une première languette de déverrouillage (46), que la première languette de déverrouillage (46) est formée en repliant un premier des deux coins secondaires (42, 44) de la première position de pliage à une deuxième position de pliage autour d'une ligne de pliage secondaire (48) et que la ligne de pliage secondaire (48) s'étend parallèlement ou quasiment parallèlement à la ligne de pliage principale (24) et entre la ligne de pliage principale (24) et les coins secondaires (42, 44) dans la première position de pliage.

4. Dispositif d'emballage médical selon la revendication 3, **caractérisé en ce qu'**un premier des deux coins principaux (34, 36) est replié de la deuxième position de pliage vers une troisième position de pliage autour d'une première des deux lignes de pliage latérales (26, 28) en direction d'un deuxième des deux coins principaux (34, 36) et que le deuxième des deux coins principaux (34, 36) est replié de la troisième position de pliage vers une quatrième position de pliage autour d'une deuxième des deux lignes de pliage latérales (26, 28) en direction de la première des deux lignes de pliage latérales (26, 28).

5. Dispositif d'emballage médical selon l'une des revendications précédentes, **caractérisé en ce que** le dispositif de sécurité anti-déploiement (68) comprend au moins un élément de sécurité (70, 170) pour maintenir le dispositif d'emballage (10) en position d'insertion, où en particulier
a) le dispositif d'emballage (10) définit au moins une première zone de surface de sécurité (72, 172) et au moins une deuxième zone de surface de sécurité (74, 174), la première zone de surface de sécurité (72, 172) et la deuxième zone de surface de sécurité (74, 174) étant différentes des deux zones de surface de feuille de matériau plat de l'espace de réception (20, 22) qui sont adjacentes l'une à l'autre en position d'insertion, la première zone de surface de sécurité (72, 172) et la deuxième zone de surface de sécurité (74, 174) étant adjacentes l'une à l'autre dans la position d'insertion et l'au moins un élément de sécurité (70, 170) reliant l'au moins une première zone de surface de sécurité (72, 172) et l'au moins une deuxième zone de surface de sécurité (74, 174) par adhérence et/ou par liaison de matière, en particulier par collage et/ou soudage,
et/ou
b) l'au moins une première zone de surface de sécurité (72, 172) et l'au moins une deuxième zone de surface de sécurité (74, 174) qui lui est associée étant entourées par la même surface latérale de matériau plat (30, 32) ou par des surfaces latérales de matériau plat différentes (30, 32)
et/ou
c) le ou les éléments de sécurité (70, 170) étant formés par un point de soudure, par une colle ou par un élément adhésif (90), l'élément adhésif (90), en particulier, présentant notamment deux surfaces adhésives (92, 94) opposées l'une à l'autre et l'une des deux surfaces adhésives (92, 94) étant en contact avec au moins une première zone de surface de sécurité (72, 172) dans la position d'insertion et l'autre des deux surfaces adhésives (92, 94) étant en contact avec au moins une deuxième zone de surface de sécurité (72, 172),
et/ou
d) le ou les éléments de sécurité (70, 170) étant positionnés dans une zone du dispositif d'emballage (10) dans laquelle, en position d'insertion, au moins deux couches formées par pliage de la feuille de matériau plat (18) sont superposées.

6. Dispositif d'emballage médical selon l'une des revendications précédentes, **caractérisé en ce que** le dispositif d'emballage (10) comprend au moins un élément de fermeture (102, 104) pour fermer le dispositif d'emballage (10) dans une position d'emballage dans laquelle l'ouverture d'insertion (16) est fermée en repliant le deuxième coin secondaire (44) autour d'une ligne de pliage de fermeture (100) en direction du premier coin secondaire (42).

7. Dispositif d'emballage médical selon l'une des revendications précédentes, **caractérisé en ce que**
a) la feuille de matériau plat (18) est constituée d'un tissu, d'un matériau non tissé, en particulier d'un matériau non tissé en matière plastique, ou d'un papier d'emballage, en particulier d'un papier crépon,
et/ou
b) le dispositif d'emballage (10) est conçu sous la forme d'un produit jetable
et/ou
c) le dispositif d'emballage (10) est conçu de manière à pouvoir être stérilisé, en particulier par stérilisation à la vapeur chaude,
et/ou
d) le dispositif d'emballage (10) est constitué d'une feuille de matériau plat (18) d'une seule pièce, en particulier monolithique.

8. Système d'emballage médical (116), en particulier stérile, pour paniers perforés (12) comprenant une pluralité de dispositifs d'emballage médical (10) selon l'une des revendications précédentes,
où en particulier
a) au moins deux des dispositifs d'emballage médical (10) se distinguent l'un de l'autre par leur forme et/ou leur taille et/ou par le matériau formant la feuille de matériau plat (18),
et/ou
b) le système d'emballage (116) comprend un suremballage (118) pour la majorité des dispositifs d'emballage (10), en particulier sous la forme d'un dispositif de distribution (120) avec une ouverture de prélèvement (122) à travers laquelle les dispositifs d'emballage (10) peuvent être prélevés individuellement du dispositif de distribution (120),
et/ou
c) le système d'emballage (116) comprend au moins un panier perforé (12).

9. Procédé de fabrication d'un dispositif d'emballage médical (10) pour l'emballage stérile d'un panier perforé (12), le dispositif d'emballage (10) étant conçu avec un espace de réception (14) pour héberger un panier perforé (12) dans une position d'emballage et une ouverture d'insertion (16) pour insérer un panier perforé (12) dans l'espace de réception (14), l'ouverture d'insertion (16) étant ouverte dans une position d'insertion, le dispositif d'emballage (10) étant formé par pliage multiple d'une feuille de matériau plat (18) non pliée dans une position initiale, l'espace de réception (14) présentant dans la position d'insertion deux zones de surface de feuille de matériau plat d'espace de réception (20, 22) adjacentes l'une à l'autre, qui sont délimitées par au moins trois lignes de pliage (24, 26, 28) de la feuille de matériau plat (18), de telle sorte que l'espace de réception (14) est fermé de tous les côtés à l'exception de l'ouverture d'insertion (16), **caractérisé en ce que** le dispositif d'emballage (10) est bloqué dans la position d'insertion pour empêcher un dépliage complet et le ramener dans la position initiale.

10. Procédé selon la revendication 9, **caractérisé en ce que**
a) la feuille de matériau plat (18) définit une première surface latérale de matériau plat (30) et une deuxième surface latérale de matériau plat (32), que la première surface latérale de matériau plat (30) et la deuxième surface latérale de matériau plat (32) sont orientées dans des directions opposées l'une de l'autre dans la position initiale et que les deux zones de surface de feuille de matériau plat de l'espace de réception (20, 22) sont formées à partir de la première surface latérale de matériau plat (30) en pliant la feuille de matériau plat (18)
et/ou
b) le dispositif d'emballage (10) est formé par pliage de la feuille de matériau plat (18) le long d'au moins trois lignes de pliage (24, 26, 28), en particulier le long de trois, quatre, cinq, six ou sept lignes de pliage,
et/ou
c) une feuille de matériau plat (18) est fournie, laquelle est de forme carrée, en particulier rectangulaire, en outre en particulier voire carrée ou essentiellement carrée dans la position initiale,
et/ou
d) l'une des trois lignes de pliage au moins est réalisée sous la forme d'une ligne de pliage principale (24) qui relie entre eux deux coins principaux (34, 36) de la feuille de matériau plat (18) qui sont opposés l'un à l'autre dans la position initiale.

11. Procédé selon la revendication 9 ou 10, **caractérisé en ce que** la feuille de matériau plat (18) comprend deux coins secondaires opposés (42, 44) dans la position initiale, que les deux coins secondaires (42, 44) sont amenés par pliage de la feuille de matériau plat (18) de la position initiale autour de la ligne de pliage principale (24) dans une première position de pliage dans laquelle ils sont superposés, qu'une première languette de déverrouillage (46) du dispositif d'emballage (10) est formée en repliant un premier des deux coins secondaires (42, 44) autour d'une ligne de pliage secondaire (48) de la première position de pliage à une deuxième position de pliage et que la ligne de pliage secondaire (48) s'étend parallèlement ou quasiment parallèlement à la ligne de pliage principale (24) et entre la ligne de pliage principale (24) et les coins secondaires (42, 44) dans la première position de pliage, où en particulier
a) la première languette de déverrouillage (46) étant conçue de manière à dépasser de la ligne de pliage principale (24) dans la position d'insertion
et/ou
b) la ligne de pliage secondaire (48) étant formée avec un écart (50) par rapport à la ligne de pliage principale (24), lequel est inférieur à un écart (52) par rapport aux coins secondaires (42, 44) dans la première position de pliage,
et/ou
c) la ligne de pliage secondaire (48) étant formée de manière à délimiter l'ouverture d'insertion (16).

12. Procédé selon la revendication 11, **caractérisé en ce qu'**un premier des deux coins principaux (34, 36) est replié de la deuxième position de pliage vers une troisième position de pliage autour d'une première des deux lignes de pliage latérales (26, 28) en direction d'un deuxième des deux coins principaux (34, 36) et que le deuxième des deux coins principaux (34, 36) est replié de la troisième position de pliage vers une quatrième position de pliage autour d'une deuxième des deux lignes de pliage latérales (26, 28) en direction de la première des deux lignes de pliage latérales (26, 28),
où en particulier le dispositif d'emballage (10) étant notamment conçu de telle sorte que la quatrième position de pliage définit la position d'insertion.

13. Procédé selon la revendication 12, **caractérisé en ce qu'**une deuxième languette de déverrouillage (54) du dispositif d'emballage (10) est formée en repliant un deuxième des deux coins secondaires (42, 44) qui, dans la position d'insertion, est orientée à l'opposé de l'ouverture d'insertion (16), de la quatrième position de pliage à une cinquième position de pliage autour d'une ligne de pliage principale de languette de déverrouillage (56) en direction de la ligne de pliage principale (24) et en repliant le deuxième coin secondaire (44) de la cinquième position de pliage à une sixième position de pliage autour d'une ligne de pliage secondaire de languette de déverrouillage (58) dans une direction s'éloignant de la ligne de pliage principale (24).

14. Procédé selon l'une des revendications 9 à 13, **caractérisé en ce que** le dispositif d'emballage (10) est bloqué en position d'insertion par au moins un élément de sécurité (70, 170),
où en particulier
a) le dispositif d'emballage (10) définissant au moins une première zone de surface de sécurité (72, 172) et au moins une deuxième zone de surface de sécurité (74, 174), la première zone de surface de sécurité (72, 172) et la deuxième zone de surface de sécurité (74, 174) étant différentes des deux zones de surface de matériau plat de l'espace de réception (20, 22) qui sont adjacentes l'une à l'autre en position d'insertion, la première zone de surface de sécurité (72, 172) et la deuxième zone de surface de sécurité (74, 174) étant adjacentes l'une à l'autre en position d'insertion et, avec l'au moins un élément de sécurité (70, 170), l'au moins une première zone de surface de sécurité (72, 172) et l'au moins une deuxième zone de surface de sécurité (74, 174) étant reliés entre eux par adhérence et/ou par liaison de matière, en particulier par collage et/ou par soudage,
où, en outre en particulier le dispositif d'emballage (10) étant conçu de telle sorte que l'au moins une première zone de surface de sécurité (72, 172) et l'au moins une deuxième zone de surface de sécurité (74, 174) qui lui est associée soient entourées par la même surface latérale de matériau plat (30, 32) ou par des surfaces latérales de matériau plat différentes (30, 32),
et/ou
b) le ou les éléments de sécurité (70, 170) sont formés par un point de soudure, par une colle ou par un élément adhésif (90), l'élément adhésif (90) présentant notamment deux surfaces adhésives (92, 94) opposées et étant appliqué de telle manière, en particulier dans la position initiale, de telle sorte que dans la position d'insertion, l'une des deux surfaces adhésives (92, 94) soit en contact avec au moins une première zone de surface de sécurité (72, 172) et que l'autre des deux surfaces adhésives (92, 94) soit en contact avec au moins une deuxième zone de surface de sécurité (74, 174),
et/ou
c) l'au moins un élément de sécurité (70, 170) est positionné dans une zone du dispositif d'emballage (10) dans laquelle, en position d'insertion, au moins deux couches formées par pliage de la feuille de matériau plat (18) sont superposées.

15. Procédé selon l'une des revendications 9 à 14, **caractérisé en ce que**
a) la feuille de matériau plat (18) est constituée d'un tissu, d'un matériau non tissé, en particulier d'un matériau non tissé en matière plastique, ou d'un papier d'emballage, en particulier d'un papier crépon,
et/ou
b) le dispositif d'emballage (10) est conçu sous la forme d'un produit jetable
et/ou
c) le dispositif d'emballage (10) est conçu de manière à pouvoir être stérilisé, en particulier à la vapeur chaude,
et/ou
d) le dispositif d'emballage (10) est constitué d'une feuille de matériau plat (18) d'une seule pièce, en particulier monolithique.
